(19) Europäisches Patentamt
European Patent Office
Office européen des brevets

(11) Veröffentlichungsnummer : **0 530 149 A1**

# (12) EUROPÄISCHE PATENTANMELDUNG

(21) Anmeldenummer : **92810641.8**

(22) Anmeldetag : **21.08.92**

(51) Int. Cl.[5] : **C07D 403/12,** C07D 405/12, C07D 409/12, C07D 333/58, C07D 333/62, A01N 43/54

(30) Priorität : **30.08.91 CH 2549/91**

(43) Veröffentlichungstag der Anmeldung :
**03.03.93 Patentblatt 93/09**

(84) Benannte Vertragsstaaten :
**AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Walter, Harald, Dr.**
**Rufacherstrasse 20**
**CH-4055 Basel (CH)**
Erfinder : **Havenhand, Mark, Dr.**
**Tellstrasse 44**
**CH-4053 Basel (CH)**

(54) **Schädlingsbekämpfungsmittel.**

(57) Verbindungen der Formel

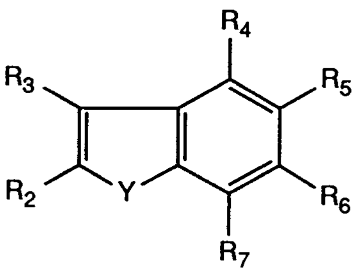

(I),

worin bedeuten :
einer der Reste $R_2$, $R_3$, $R_4$, $R_5$, $R_6$ und $R_7$ eine Gruppe der Formel

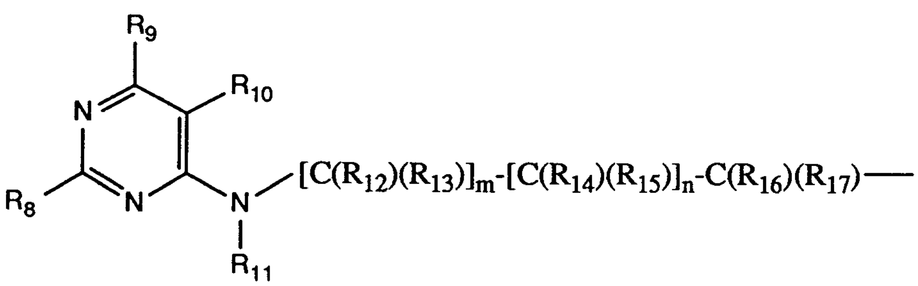

(Ia);

die anderen fünf der Reste $R_2$, $R_3$, $R_4$, $R_5$, $R_6$ und $R_7$, unabhängig voneinander, Wasserstoff, Halogen, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, Halogen-$C_1$-$C_8$-alkoxy, $C_1$-$C_8$-Alkylthio, Halogen-$C_1$-$C_8$-Alkylthio, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten substituiert sind ;
Y ein Sauerstoffatom, ein Schwefelatom oder eine Gruppe der Formel $NR_1$ (Ib) ; worin
$R_1$ Wasserstoff, $C_1$-$C_8$-Alkyl, $C_1$-$C_8$-Alkanoyl, $C_1$-$C_8$-Alkansulfonyl, Halogen-$C_1$-$C_8$-alkansulfonyl, Cyano-$C_1$-$C_8$-alkansulfonyl oder Phenylsulfonyl bedeutet, dessen Phenylgruppe unsubstituiert oder durch einen oder zwei Substituenten, ausgewählt aus der Gruppe bestehend aus Halogen, $C_1$-$C_4$-Alkyl, Nitro und Cyano, substituiert ist ;
m 0 oder 1 ; und
n 0 oder 1 ; und

EP 0 530 149 A1

$R_8$ bis $R_{20}$ die hierin gegebene Bedeutung haben, und gegebenenfalls Tautomere davon, jeweils in freier Form oder in Salzform, können als agrochemische Wirkstoffe verwendet werden.

Die Erfindung betrifft Verbindungen der Formel

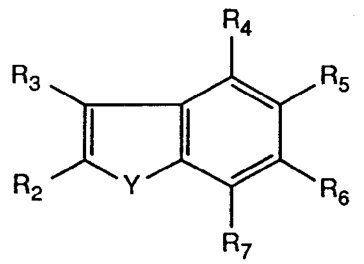

(I),

worin bedeuten:
einer der Reste $R_2$, $R_3$, $R_4$, $R_5$, $R_6$ und $R_7$ eine Gruppe der Formel

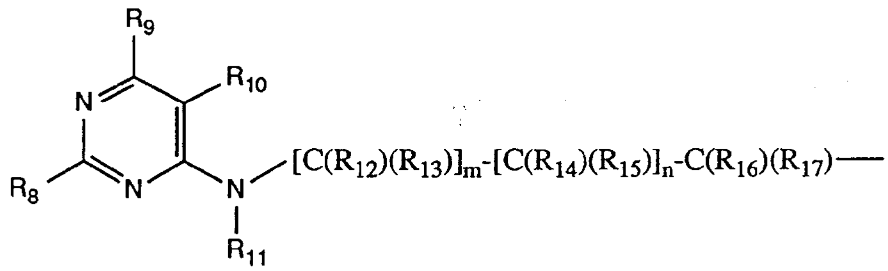

(Ia);

die anderen fünf der Reste $R_2$, $R_3$, $R_4$, $R_5$, $R_6$ und $R_7$, unabhängig voneinander, Wasserstoff, Halogen, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, Halogen-$C_1$-$C_8$-alkoxy, $C_1$-$C_8$-Alkylthio, Halogen-$C_1$-$C_8$-alkylthio, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_4$-Alkyl und $C_1$-$C_4$-Alkoxy, substituiert sind, mit der Massgabe, dass von den fünf Resten aus der Gruppe der Reste $R_2$, $R_3$, $R_4$, $R_5$, $R_6$ und $R_7$, die von der Gruppe Ia verschieden sind, mindestens zwei Reste für Wasserstoff stehen und mindestens vier Reste von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind;
Y ein Sauerstoffatom, ein Schwefelatom oder eine Gruppe der Formel $NR_1$ (Ib);
$R_1$ Wasserstoff, $C_1$-$C_8$-Alkyl, $C_1$-$C_8$-Alkanoyl, $C_1$-$C_8$-Alkansulfonyl, Halogen-$C_1$-$C_8$-alkansulfonyl, Cyano-$C_1$-$C_8$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_4$-Alkyl, Nitro und Cyano, substituiert ist;
$R_6$ Wasserstoff, $C_1$-$C_8$-Alkyl, $C_1$-$C_8$-Alkoxy oder $C_1$-$C_8$-Alkylthio;
$R_9$ Wasserstoff, $C_1$-$C_8$-alkyl, Halogen-$C_1$-$C_8$-alkyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkansulfinyl-$C_1$-$C_4$-alkyl, $C_1$-$C_4$-Alkansulfonyl-$C_1$-$C_4$-alkyl, $C_2$-$C_8$-Alkenyl, Halogen-$C_2$-$C_8$-alkenyl, $C_2$-$C_8$-Alkinyl, $C_3$-$C_8$-Cycloalkyl oder Halogen;
$R_{10}$ Wasserstoff, Hydroxy, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, $C_1$-$C_8$-Alkylthio, $C_1$-$C_8$-Alkansulfinyl, $C_1$-$C_8$-Alkansulfonyl, Halogen, Nitro, Cyano, Amino, eine Gruppe der Formel $N(H)R_{18}$ (Ic), eine Gruppe der Formel $N(R_{18})R_{19}$ (Id) oder eine Gruppe der Formel $N{=}C(R_{19})R_{20}$ (Ie);
$R_{11}$ Wasserstoff, $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_4$-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind;
$R_{12}$, $R_{13}$, $R_{14}$, $R_{15}$, $R_{16}$ und $R_{17}$, unabhängig voneinander, Wasserstoff, $C_1$-$C_8$-Alkyl, Halogen-$C_1$-$C_8$-alkyl, $C_1$-$C_4$-Alkoxy-$C_1$-$C_4$-alkyl oder $C_3$-$C_8$-Cycloalkyl;
$R_{18}$ $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_4$-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind;

$R_{19}$ $C_1$-$C_8$-Alkyl;
$R_{20}$ Wasserstoff oder $C_1$-$C_8$-Alkyl;
m 0 oder 1; und
n 0 oder 1;
und gegebenenfalls Tautomere davon, jeweils in freier Form oder in Salzform, ein Verfahren zur Herstellung und die Verwendung dieser Verbindungen und Tautomere sowie Schädlingsbekämpfungsmittel, deren Wirkstoff aus diesen Verbindungen und Tautomeren, jeweils in freier Form oder in agrochemisch verwendbarer Salzform, ausgewählt ist, und ein Verfahren zur Herstellung und die Verwendung dieser Mittel.

Die Verbindungen I können teilweise als Tautomere vorliegen. Bedeutet z. B. der Rest $R_{10}$ in der Gruppe Ia Hydroxy, können entsprechende Verbindungen im Gleichgewicht mit den tautomeren Oxo-Derivaten, d. h. den entsprechenden Pyrimid-5-onen, stehen. Demgemäss sind unter den Verbindungen nachstehend gegebenenfalls auch entsprechende Tautomere zu verstehen, auch wenn letztere nicht in jedem Fall speziell erwählt werden.

Die Verbindungen I und gegebenenfalls ihre Tautomeren können als, insbesondere agrochemisch verwendbare, Salze vorliegen. Da die Verbindungen I mindestens ein basisches Zentrum aufweisen, können sie z. B. Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$-$C_4$-Alkancarbonsäuren, z.B. Essigsäure, wie gegebenenfalls ungesättigten Dicarbonsäuren, z.B. Oxal-, Malon-, Malein-, Fumar- oder Phthalsäure, wie Hydroxycarbonsäuren, z.B. Ascorbin-, Milch-; Äpfel-, Wein- oder Zitronensäure, oder wie Benzoesäure, oder mit organischen Sulfonsäuren, wie gegebenenfalls, z.B. durch Halogen, substituierten $C_1$-$C_4$-Alkan- oder Aryl-sulfonsäuren, z.B. Methan- oder p-Toluolsulfonsäure, gebildet. Ferner können Verbindungen I mit mindestens einer aciden Gruppe Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z.B. Ethyl-, Diethyl-, Triethyl- oder Dimethyl-propyl-amin, oder einem Mono-, Di- oder Trihydroxyniederalkylamin, z.B. Mono-, Di- oder Triethanolamin. Weiterhin können gegebenenfalls entsprechende innere Salze gebildet werden. Umfasst sind ferner für agrochemische Verwendungen nicht geeignete Salze, die beispielsweise für die Isolierung bzw. Reinigung von freien Verbindungen I oder deren agrochemisch verwendbaren Salzen eingesetzt werden. Infolge der engen Beziehung zwischen den Verbindungen I in freier Form und in Form ihrer Salze sind vorstehend und nachfolgend unter den freien Verbindungen I bzw. ihren Salzen sinn- und zweckgemäss gegebenenfalls auch die entsprechenden Salze bzw. die freien Verbindungen I zu verstehen. Entsprechendes gilt für Tautomere von Verbindungen I und deren Salze.

Die vor- und nachstehend verwendeten Allgemeinbegriffe haben, sofern nicht abweichend definiert, die nachfolgend aufgeführten Bedeutungen.

Halogen ist Fluor, Chlor, Brom oder Iod.

Kohlenstoffhaltige Gruppen und Verbindungen enthalten, sofern nicht abweichend definiert, jeweils 1 bis und mit 8, vorzugsweise 1 bis und mit 4, insbesondere 1 oder 2, Kohlenstoffatome.

Alkyl - als Gruppe per se sowie als Strukturelement von anderen Gruppen und Verbindungen, wie von Halogenalkyl, Alkoxy, Halogenalkoxy, Alkoxyalkyl, Alkylthio, Cyanoalkylthio, Halogenalkylthio, Alkylthioalkyl, Alkansulfinyl, Alkansulfinylalkyl, Alkansulfonyl, Cyanoalkansulfonyl, Halogenalkansulfonyl und Alkansulfonylalkyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl, Hexyl, Heptyl oder Octyl, oder verzweigt, z. B. Isopropyl, Isobutyl, sek.-Butyl, tert.-Butyl, Isopentyl, Neopentyl oder Isooctyl.

Alkenyl - als Gruppe per se sowie als Strukturelement von anderen Gruppen und Verbindungen, wie von Halogenalkenyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig, z. B. Ethenyl, Propen-1-yl, But-1-en-1-yl, Pent-2-en-1-yl oder Oct-3-en-1-yl, oder verzweigt, z.B. Propen-2-yl, But-1-en-2-yl oder Oct-2-en-4-yl.

Alkinyl ist, jeweils unter gebührender Berücksichtigung der von Fall zu Fall umfassten Anzahl Kohlenstoffatome, entweder geradkettig, z. B. Ethinyl, Propin-1-yl, But-1-in-1-yl, Pent-2-in-1-yl oder Oct-3-in-1-yl, oder verzweigt, z. B. Propin-2-yl, But-1-in-2-yl oder Oct-2-in-4-yl.

Alkanoyl ist, jeweils unter gebührender Berücksichtigung der von Fall zu Fall umfassten Anzahl Kohlenstoffatome, entweder geradkettig oder verzweigt, z. B. Formyl, Acetyl, Propionyl, Butyryl, Pivaloyl oder Octanoyl.

Cycloalkyl ist, jeweils unter gebührender Berücksichtigung der von Fall zu Fall umfassten Anzahl Kohlenstoffatome, Cyclopropyl, Cyclobutyl, Cyclopentyl, Cyclohexyl, Cycloheptyl oder Cyclooctyl.

In Halogen-substituierten kohlenstoffhaltigen Gruppen und Verbindungen, wie Halogenalkyl, Halogenalkenyl, Halogenalkoxy, Halogenalkylthio und Halogenalkansulfonyl, ist mindestens eines der in der zugrundeliegenden nicht-halogenierten Grundstruktur vorhandenen Wasserstoffatome durch ein Halogenatom ersetzt; es können jedoch auch zwei oder mehr als zwei, z. B. - im Falle einer Perhalogenierung - alle, der in der zugrundeliegenden nicht-halogenierten Grundstruktur vorhandenen Wasserstoffatome unabhängig voneinander durch Halogenatome ersetzt sein. Beispielhaft erwähnt seien $-CH_2F$, $-CHF_2$, $-CF_3$, $-CH_2Cl$, $-CHCl_2$, $-CCl_3$, $-CCl_2CCl_3$, $-CH_2Br$, $-CH_2CH_2Br$, -CHBrCl, $-CCl=CCl_2$, $-OCH_2F$, $-SCHCl_2$ und $-SO_2CF_3$.

In Alkoxy-, Alkylthio-, Alkansulfinyl-, Alkansulfonyl- bzw. Cyano-substituierten kohlenstoffhaltigen Gruppen und Verbindungen, wie Alkoxyalkyl, Alkylthioalkyl, Alkansulfinylalkyl, Alkansulfonylalkyl, Cyanoalkylthio und Cyanoalkansulfonyl, ist eines der in der zugrundeliegenden unsubstituierten Grundstruktur vorhandenen Wasserstoffatome durch Alkoxy, Alkylthio, Alkansulfinyl, Alkansulfonyl bzw. Cyano ersetzt.

Bevorzugt sind im Rahmen der Erfindung Verbindungen der Formel I, worin bedeuten: einer der Reste $R_2$, $R_3$, $R_4$, $R_5$, $R_6$ und $R_7$ eine Gruppe Ia;

die anderen fünf der Reste $R_2$, $R_3$, $R_4$, $R_6$, $R_6$ und $R_7$, unabhängig voneinander, Wasserstoff, Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_4$-Alkoxy, Halogen-$C_1$-$C_4$-alkoxy mit 1, 2 oder 3 Halogenatomen, $C_1$-$C_4$-Alkylthio, Halogen-$C_1$-$C_4$-alkylthio mit 1, 2 oder 3 Halogenatomen, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_2$-Alkyl und $C_1$-$C_2$-Alkoxy, substituiert sind, mit der Massgabe, dass von den fünf Resten aus der Gruppe der Reste $R_2$, $R_3$, $R_4$, $R_6$, $R_6$ und $R_7$, die von der Gruppe Ia verschieden sind, mindestens zwei Reste für Wasserstoff stehen und mindestens vier Reste von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind;

Y ein Sauerstoffatom, ein Schwefelatom oder eine Gruppe Ib;

$R_1$ Wasserstoff, $C_1$-$C_4$-Alkyl, $C_1$-$C_4$-Alkanoyl, $C_1$-$C_4$-Alkansulfonyl, Halogen-$C_1$-$C_4$-alkansulfonyl mit 1, 2 oder 3 Halogenatomen, Cyano-$C_1$-$C_4$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;

$R_6$ Wasserstoff, $C_1$-$C_4$-Alkyl, $C_1$-$C_4$-Alkoxy oder $C_1$-$C_4$-Alkylthio;

$R_9$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$-$C_4$-alkyl, $C_1$-$C_2$-Alkansulfonyl-$C_1$-$C_4$-alkyl, $C_2$-$C_4$-Alkenyl, Halogen-$C_2$-$C_4$-alkenyl mit 1, 2 oder 3 Halogenatomen, $C_2$-$C_4$-Alkinyl, $C_3$-$C_7$-Cycloalkyl oder Halogen;

$R_{10}$ Wasserstoff, Hydroxy, $C_1$-$C_4$-Alkyl, Halogen-$C_1$-$C_4$-alkyl mit 1, 2 oder 3 Halogenatomen, $C_1$-$C_4$-Alkoxy-$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro, Cyano, Amino, eine Gruppe Ic, eine Gruppe Id oder eine Gruppe Ie;

$R_{11}$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;

$R_{12}$, $R_{13}$, $R_{14}$, $R_{15}$, $R_{16}$ und $R_{17}$, unabhängig voneinander, Wasserstoff, $C_1$-$C_4$-Alkyl, Halogen-$C_1$-$C_4$-alkyl mit 1, 2 oder 3 Halogenatomen, $C_1$-$C_4$-Alkoxy-$C_1$-$C_4$-alkyl oder $C_3$-$C_7$-Cycloalkyl;

$R_{18}$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;

$R_{19}$ $C_1$-$C_4$-Alkyl;

$R_{20}$ Wasserstoff oder $C_1$-$C_4$-Alkyl;

m 0 oder 1; und

n 0 oder 1;

und gegebenenfalls Tautomere davon, jeweils in freier Form oder in Salzform.

Besonders bevorzugt sind im Rahmen der Erfindung Verbindungen der Formel I, worin bedeuten: einer der Reste $R_2$, $R_3$, $R_6$ und $R_6$ eine Gruppe Ia;

die anderen drei der Reste $R_2$, $R_3$, $R_6$ und $R_6$ und die Reste $R_4$ und $R_7$, unabhängig voneinander, Wasserstoff, Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_4$-

Alkoxy, Halogen-$C_1$-$C_4$-alkoxy mit 1, 2 oder 3 Halogenatomen, $C_1$-$C_4$-Alkylthio, Halogen-$C_1$-$C_4$-alkylthio mit 1, 2 oder 3 Halogenatomen, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewähnt aus der Gruppe bestehend aus Halogen, $C_1$-$C_2$-Alkyl und $C_1$-$C_2$-Alkoxy, substituiert sind, mit der Massgabe, dass von den fünf Resten aus der Gruppe der Reste $R_2$, $R_3$, $R_4$, $R_5$, $R_6$ und $R_7$, die von der Gruppe Ia verschieden sind, mindestens zwei Reste für Wasserstoff stehen und mindestens vier Reste von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind;
Y ein Sauerstoffatom, ein Schwefelatom oder eine Gruppe Ib;
$R_1$ Wasserstoff, $C_1$-$C_4$-Alkyl, $C_1$-$C_4$-Alkanoyl, $C_1$-$C_4$-Alkansulfonyl, Halogen-$C_1$-$C_4$-alkansulfonyl mit 1, 2 oder 3 Halogenatomen, Cyano-$C_1$-$C_4$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;
$R_8$ Wasserstoff, $C_1$-$C_4$-Alkyl, $C_1$-$C_4$-Alkoxy oder $C_1$-$C_4$-Alkylthio;
$R_9$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$ -$C_4$-alkyl, $C_1$-$C_2$-Alkansulfonyl-$C_1$-$C_4$-alkyl, $C_2$-$C_4$-Alkeny l, Halogen-$C_2$-$C_4$-alkenyl mit 1, 2 oder 3 Halogenatomen, $C_2$-$C_4$-Alkinyl, $C_3$-$C_7$-Cycloalkyl oder Halogen;
$R_{10}$ Wasserstoff, Hydroxy, $C_1$-$C_4$-Alkyl, Halogen-$C_1$-$C_4$-alkyl mit 1, 2 oder 3 Halogenatomen, $C_1$-$C_4$-Alkoxy-$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro, Cyano, Amino, eine Gruppe Ic, eine Gruppe Id oder eine Gruppe Ie;
$R_{11}$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_{12}$, $R_{13}$, $R_{14}$, $R_{15}$, $R_{16}$ und $R_{17}$, unabhängig voneinander, Wasserstoff, $C_1$-$C_4$-Alkyl, Halogen-$C_1$-$C_4$-alkyl mit 1, 2 oder 3 Halogenatomen, $C_1$-$C_4$-Alkoxy-$C_1$-$C_4$-alkyl oder $C_3$-$C_7$-Cycloalkyl;
$R_{18}$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_{19}$ $C_1$-$C_4$-Alkyl;
$R_{20}$ Wasserstoff oder $C_1$-$C_4$-Alkyl;
m 0 oder 1; und
n 0 oder 1;
und gegebenenfalls Tautomere davon, jeweils in freier Form oder in Salzform.

Insbesondere bevorzugt sind im Rahmen der Erfindung Verbindungen der Formel I, worin bedeuten:
einer der Reste $R_2$, $R_3$, $R_8$ und $R_8$ eine Gruppe Ia;
die anderen drei der Reste $R_2$, $R_3$, $R_8$ und $R_6$ und die Reste $R_4$ und $R_7$, unabhängig voneinander, Wasserstoff, Halogen, $C_1$-$C_4$-Alkyl, $C_1$-$C_4$-Alkoxy oder unsubstituiertes Phenyl, mit der Massgabe, dass von den fünf Resten aus der Gruppe der Reste $R_2$, $R_3$, $R_4$, $R_8$, $R_6$ und $R_7$, die von der Gruppe Ia verschieden sind, mindestens zwei Reste für Wasserstoff stehen und mindestens vier Reste von unsubstituiertem Phenyl verschieden sind;
Y ein Sauerstoffatom, ein Schwefelatom oder eine Gruppe Ib;
$R_1$ Wasserstoff, $C_1$-$C_4$-Alkyl, $C_1$-$C_4$-Alkanoyl oder unsubstituiertes Phenylsulfonyl;
$R_8$ Wasserstoff oder $C_1$-$C_4$-Alkyl;
$R_9$ $C_1$-$C_4$-Alkyl, $C_3$-$C_4$-Cycloalkyl oder Halogen;
$R_{10}$ $C_1$-$C_4$-Alkyl, Halogen oder Nitro;
$R_{11}$, $R_{12}$, $R_{13}$, $R_{14}$, $R_{15}$ und $R_{16}$ jeweils Wasserstoff;
$R_{17}$ Wasserstoff oder $C_1$-$C_4$-Alkyl;
m 0 oder 1; und
n 0 oder 1;
in freier Form oder in Salzform.

In besonderer Weise bevorzugt sind im Rahmen der Erfindung Verbindungen der Formel I, worin bedeuten:
einer der Reste $R_2$, $R_3$ und $R_8$ eine Gruppe Ia;

die beiden anderen der Reste $R_2$, $R_3$ und $R_6$ und die Reste $R_4$, $R_6$ und $R_7$, unabhängig voneinander, Wasserstoff, Halogen, $C_1$-$C_4$-Alkyl oder $C_1$-$C_4$-Alkoxy, mit der Massgabe, dass von den fünf Resten aus der Gruppe der Reste $R_2$, $R_3$, $R_4$, $R_6$, $R_6$ und $R_7$, die von der Gruppe Ia verschieden sind, mindestens drei Reste für Wasserstoff stehen;
Y ein Sauerstoffatom, ein Schwefelatom oder eine Gruppe Ib;
$R_1$ Wasserstoff oder $C_1$-$C_4$-Alkyl;
$R_6$ Wasserstoff;
$R_9$ $C_1$-$C_4$-Alkyl;
$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

Besonders bevorzugt sind im Rahmen der Erfindung Verbindungen der Formel I, worin bedeuten:

(a) $R_2$ eine Gruppe Ia, $R_3$ Wasserstoff oder $C_1$-$C_4$-Alkyl, $R_4$ Wasserstoff, $R_6$ Wasserstoff, Halogen oder $C_1$-$C_4$-Alkoxy, $R_6$ und $R_7$ jeweils Wasserstoff, mit der Massgabe, dass von den fünf Resten $R_3$, $R_4$, $R_5$, $R_6$ und $R_7$ mindestens vier Reste für Wasserstoff stehen, Y ein Sauerstoffatom, ein Schwefelatom oder eine Gruppe Ib und $R_1$ Wasserstoff oder $C_1$-$C_4$-Alkyl, oder

(b) $R_2$ Wasserstoff, $R_3$ eine Gruppe Ia, $R_4$, $R_6$, $R_6$ und $R_7$ jeweils Wasserstoff, Y eine Gruppe Ib und $R_1$ Wasserstoff oder $C_1$-$C_4$-Alkyl; oder

(c) $R_2$ und $R_3$, unabhängig voneinander, $C_1$-$C_4$-Alkyl, $R_4$ und $R_6$ jeweils Wasserstoff, $R_6$ eine Gruppe Ia, $R_7$ Wasserstoff, Y ein Sauerstoffatom oder eine Gruppe Ib und $R_1$ Wasserstoff; sowie jeweils

$R_6$ Wasserstoff;
$R_9$ $C_1$-$C_4$-Alkyl;
$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

Ganz besonders bevorzugt sind im Rahmen der Erfindung Verbindungen der Formel I, worin bedeuten:

(d) $R_2$ eine Gruppe Ia, $R_6$ Wasserstoff und entweder $R_3$ Wasserstoff oder $C_1$-$C_4$-Alkyl, $R_6$ Wasserstoff und Y ein Schwefelatom oder $R_3$ Wasserstoff, $R_5$ Halogen oder $C_1$-$C_4$-Alkoxy und Y ein Sauerstoffatom; oder

(e) $R_2$ und $R_3$, unabhängig voneinander, $C_1$-$C_4$-Alkyl, $R_5$ Wasserstoff, $R_6$ eine Gruppe Ia und Y ein Sauerstoffatom; sowie jeweils

$R_4$, $R_7$ und $R_6$ jeweils Wasserstoff;
$R_9$ $C_1$-$C_4$-Alkyl;
$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

Speziell bevorzugt sind im Rahmen der Erfindung Verbindungen der Formel I, worin bedeuten:
entweder $R_3$ $C_1$-$C_4$-Alkyl, $R_6$ Wasserstoff und Y ein Schwefelatom oder $R_3$ Wasserstoff, $R_5$ $C_1$-$C_4$-Alkoxy und Y ein Sauerstoffatom; sowie jeweils
$R_2$ eine Gruppe Ia;
$R_4$, $R_6$, $R_7$ und $R_8$ jeweils Wasserstoff;
$R_9$ $C_1$-$C_4$-Alkyl;
$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

Ganz speziell bevorzugt sind im Rahmen der Erfindung Verbindungen der Formel I, worin bedeuten:
Y ein Schwefelatom;
$R_2$ eine Gruppe Ia;
$R_3$ $C_1$-$C_4$-Alkyl;
$R_4$, $R_6$, $R_6$, $R_7$ und $R_8$ jeweils Wasserstoff;
$R_9$ $C_1$-$C_4$-Alkyl;

$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

In erster Linie bevorzugt sind im Rahmen der Erfindung die in den Beispielen H-1 bis H-9.6 genannten Verbindungen der Formel I, in freier Form oder in Salzform.

Namentlich bevorzugt sind im Rahmen der Erfindung folgende Verbindungen,
(d,l)-2-[1-(5-Chlor-6-ethyl-pyrimidin-4-ylamino)ethyl]-3-methyl-benzo[b]thiophen,
(d,l)-2-[1-(5-Chlor-6-ethyl-pyrimidin-4-ylamino)ethyl]-5-methoxy-benzo[b]furan,
(d,l)-2-[1-(5-Chlor-6-ethyl-pyrimidin-ylamino)ethyl]-benzo[b]thiophen,
(d,l)-2-[1-(5-Chlor-6-ethyl-pyrimidin-4-ylamino)propyl]-benzo[b]thiophen,
(-)-2-[1-(5-Chlor-6-ethyl-pyrimidin-4-ylamino)propyl]-benzo[b]thiophen,
jeweils in freier Form oder in Salzform.

Als weiterer Gegenstand der Erfindung ist das Verfahren zur Herstellung der Verbindungen der Formel I und gegebenenfalls ihrer Tautomeren, jeweils in freier Form oder in Salzform, z. B. dadurch gekennzeichnet, dass man

a) zur Herstellung einer Verbindung der Formel I, worin $R_{11}$ Wasserstoff, $C_1$-$C_8$-Alkyl oder Benzyl ist, oder gegebenenfalls eines Tautomeren davon, jeweils in freier Form oder in Salzform, eine Verbindung der Formel

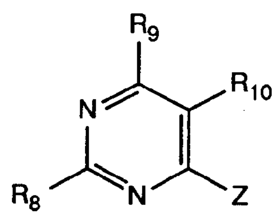

(II),

worin $R_8$, $R_9$ und $R_{10}$ die für die Formel I angegebenen Bedeutungen haben und Z ein leicht abspaltbarer nucleofuger Rest ist, oder gegebenenfalls ein Tautomeres davon mit einer Verbindung der Formel

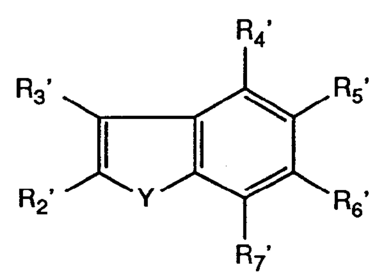

(III),

worin Y die für die Formel I angegebene Bedeutung hat und mit einer einzigen Ausnahme $R_2'$ für $R_2$, $R_3'$ für $R_3$, $R_4'$ für $R_4$, $R_8'$ für $R_5$, $R_8'$ für $R_6$ und $R_7'$ für $R_7$, wobei $R_2$, $R_3$, $R_4$, $R_8$, $R_6$ und $R_7$ die für die Formel I angegebenen Bedeutungen haben, steht, wobei die erwähnte Ausnahme darin besteht, dass anstelle der in den Definitionen der Variablen $R_2$, $R_3$, $R_4$, $R_8$, $R_8$ und $R_7$ der Formel I erwähnten Gruppe Ia die Gruppe der Formel $H(R_{11}')N-[C(R_{12})(R_{13})]_m-[C(R_{14})(R_{15})]_n-C(R_{16})(R_{17})-$ (IIIa) steht, worin $R_{11}'$ Wasserstoff, $C_1$-$C_8$-Alkyl oder Benzyl ist und $R_{12}$, $R_{13}$, $R_{14}$, $R_{15}$, $R_{16}$, $R_{17}$, m und n die für die Formel I angegebenen Bedeutungen haben, oder einem Salz davon, vorzugsweise in Gegenwart einer Base, umsetzt oder

b) zur Herstellung einer Verbindung der Formel I, worin $R_{11}$ die für die Formel I angegebene Bedeutung hat, jedoch von Wasserstoff, $C_1$-$C_8$-Alkyl und Benzyl verschieden ist, oder gegebenenfalls eines Tautomeren davon, jeweils in freier Form oder in Salzform, in eine Verbindung der Formel I, worin $R_{11}$ Wasserstoff ist, oder gegebenenfalls ein Tautomeres davon, jeweils in freier Form oder in Salzform, den gewünschten, von Wasserstoff, $C_1$-$C_8$-Alkyl und Benzyl verschiedenen, Substituenten $R_{11}$ durch N-Alkanoylierung, N-Benzoylierung, N-Alkylthiolierung, N-Phenylthiolierung oder N-Benzylthiolierung einführt

und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt ein verfahrensgemäss erhältliches Gemisch von Isomeren

auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

Für vor- und nachstehend aufgeführte Ausgangsmaterialien gilt im Hinblick auf deren Tautomere bzw. Salze das vorstehend für Tautomere bzw. Salze von Verbindungen I Gesagte in analoger Weise.

Die vor- und nachstehend beschriebenen Umsetzungen werden in an sich bekannter Weise durchgeführt z. B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsmediums, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Das vor- und nachstehend aufgeführte Ausgangsmaterial, das für die Herstellung der Verbindungen I und gegebenenfalls ihrer Tautomeren, jeweils in freier Form oder in Salzform, verwendet wird, ist bekannt oder kann nach an sich bekauten Methoden, z. B. gemäss den nachstehenden Angaben, hergestellt werden.

Variante a):

Als Reste Z kommen z. B. in Frage: Fluor, Chlor, Brom, Iod, $C_1$-$C_8$-Alkylthio, wie Methylthio, Ethylthio oder Propylthio, $C_1$-$C_8$-Alkanoyloxy, wie Acetoxy, (Halogen-)$C_1$-$C_8$-Alkansulfonyloxy, wie Methansulfonyloxy, Ethansulfonyloxy oder Trifluormethansulfonyloxy, oder gegebenenfalls substituiertes Phenylsulfonyloxy, wie Benzolsulfonyloxy oder p-Toluolsulfonyloxy, ferner auch Hydroxy.

Geeignete Basen zur Erleichterung der HZ-Abspaltung sind z. B. Alkalimetall- oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, -methanolat, -carbonat, Kalium-tert.-butanolat, -carbonat, Lithiumdiisopropylamid, Kalium-bis-(trimethylsilyl)-amid, Calciumydrid, Triethylamin, Triethylendiamin, Cyclohexylamin, N-Cyclohexyl-N,N-dimethyl-amin, N,N-Diethylanilin, Pyridin, 4-(N,N-Dimethylamino)pyridin, N-Methylmorpholin, Benzyl-trimethylammoniumhydroxid sowie 1,5-Diazabicyclo[5.4.0]undec-5-en DBU) genannt.

Die Reaktionspartier können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Ketone, wie Aceton oder Methylethylketon; Alkohole, wie Methanol, Ethanol, Propanol, Butanol, Ethylenglycol oder Glycerin; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid. Auch im Ueberschuss eingesetzte Basen, wie Triethylmin, Pyridin, N-Methylmorpholin oder N,N-Diäthylanilin, können als Lösungs- oder Verdünnungsmittel dienen.

Die Umsetzung erfolgt vorteilhaft in einem Temperaturbereich von etwa 0°C bis etwa +180°C, bevorzugt von etwa +20°C bis etwa +130°C, in vielen Fällen bei der Rückflusstemperatur des verwendeten Lösungsmittels.

In einer bevorzugten Ausführungsform der Variante a) wird eine Verbindung III bei Rückflusstemperatur, in einem Alkohol, vorzugsweise in Isopropanol, und in Gegenwart eines Alkylamins, vorzugsweise in Gegenwart von Triethylamin, mit einer Verbindung II, worin Z Halogen, vorzugsweise Chlor, ist, umgesetzt.

Die Verbindungen II und gegebenenfalls ihre Tautomeren, jeweils in freier Form oder in Salzform, sind bekannt oder können in Analogie zu bekannten Verbindungen hergestellt werden; entsprechende Angaben finden sich z. B. einersets bei D. J. Brown, "The Pyrimidines", in "Heterocyclic Compounds" und anderersets in der europäischen Patentanmeldung EP-A-0,470,600.

Die Verbindungen III, in freier Form oder in Salzform, sind bekannt oder können in Analogie zu bekannten Verbindungen, z. B. wie nachfolgend beschrieben, hergestellt werden.

Beispielsweise kann man Ammoniak oder ein Amin der Formel $H_2NR_{11}'$(IIIb) in üblicher Weise in Gegenwart eines Reduktionsmittels mit einer Verbindung der Formel IIIc umsetzen, wobei die Verbindungen IIIc den Verbindungen III entsprechen, jedoch anstelle der in den Verbindungen III vorhandenen Gruppe IIIa eine Gruppe der Formel $R_{12}C(=O)$-$[C(R_{14})(R_{15})]_n$-$C(R_{16})(R_{17})$- (IIId), eine Gruppe der Formel $R_{13}C(=O)$-$[C(R_{14})(R_{15})]_n$-

$C(R_{16})(R_{17})$- (IIIe), eine Gruppe der Formel $R_{14}C(=O)-C(R_{16})(R_{17})$- (IIIf), eine Gruppe der Formel $R_{15}C(=O)-C(R_{16})(R_{17})$- (IIIg), eine Gruppe der Formel $R_{16}C(=O)$- (IIIh) oder eine Gruppe der Formel $R_{17}C(=O)$- (IIIi) aufweisen. Geeignete Reduktionsmittel sind z. B. Wasserstoff (in Gegenwart eines Hydrierungskatalysators), Zink/Salzsäure, Natriumcyanoborhydrid, Natriumborhydrid (gegebenenfalls in Gegenwart von Titantetrachlorid), Eisenpentacarbonyl/alkoholisches Kaliumhydroxid oder Ameisensäure. Man erhält so - durch formalen Ersatz der in den Gruppen IIId, IIIe, IIIf, IIIg, IIIh bzw. IIIi enthaltenen Carbonylfunktion durch ein Wasserstoffatom und eine Gruppe der Formel $H(R_{11}')N$-(IIIj) - Verbindungen III, die an dem die Gruppe (IIIj) tragenden Kohlenstoffatom mindestens ein Wasserstoffatom aufweisen. Es kann für diese Umsetzung auch eine - gegebenenfalls vorgängig hergestellte - "Kombinationsverbindung" aus dem Reduktionsmittel und der Verbindung IIIb, z. B. ein entsprechendes Ammoniumformiat oder Formamid, verwendet werden, wobei sich nach der Umsetzung dieser "Kombinationsverbindung" mit der Verbindung IIIc gegebenenfalls gebildete Primärprodukte mit einer N-formylierten Gruppe IIIj nachfolgend in üblicher Weise hydrolytisch zu den gewünschten Verbindungen III N-deformylierel lassen.

In üblicher Weise kann man auch Hydroxylamin mit einer Verbindung IIIc umsetzen, dadurch die in den Gruppen IIId, IIIe, IIIf, IIIg, IIIh bzw. IIIi enthaltene Carbonylfunktion in die (HO-N=)-Gruppe überführen und dieses Oxim zum entsprechenden Amin reduzieren, wobei als Reduktionsmittel z. B. komplexe Metallhydride, wie $LiAlH_4$, $NaBH_4/TiCl_4$, Zink/Essigsäure oder Wasserstoff (in Gegenwart eines Hydrierungskatalysators, z. B. in Gegenwart von Raney-Nickel, gegebenenfalls in Gegenwart von Ammoniak, oder Palladium auf Kohle) Verwendung finden. Man erhält so Verbindungen III, die an dem die Gruppe $H(R_{11}')N$- (IIIj) tragenden Kohlenstoffatom mindestens ein Wasserstoffatom aufweisen und in denen $R_{11}'$ Wasserstoff ist.

Weiterhin kann man in üblicher Weise in Verbindungen der Formel IIIk, die den Verbindungen III entsprechen, jedoch anstelle der in den Verbindungen III vorhandenen Gruppe IIIa eine Gruppe der Formel $NC-[C(R_{14})(R_{15}]_n-C(R_{16})(R_{17})$- (IIIl), eine Gruppe der Formel $NC-C(R_{16})(R_{17})$- (IIIm) oder die Cyanogruppe aufweisen, die Cyanogruppe in den Gruppen IIIl bzw. IIIm bzw. die Cyanogruppe, die in den Verbindungen IIIk als Ersatz für die Gruppe IIIa in den Verbindungen III steht, zur Aminomethylgruppe reduzieren, wobei als Reduktionsmittel z. B. komplexe Metallhydride, wie $LiAlH_4$, Natriumethanolat oder Wasserstoff (in Gegenwart eines Hydrierungskatalysators, z. B. in Gegenwart von Raney-Nickel oder Palladium auf Kohle) Verwendung finden. Man erhält so Verbindungen III, die an dem die Gruppe $H(R_{11}')N$- (IIIj) tragenden Kohlenstoffatom zwei Wasserstoffatome aufweisen und in denen $R_{11}'$ Wasserstoff ist.

Des weiteren kann man auch in üblicher Weise in Verbindungen der Formel IIIn, die den Verbindungen III entsprechen, jedoch anstelle der in den Verbindungen III vorhandenen Gruppe IIIa eine Gruppe der Formel $HN(R_{11}')-C(=O)-[C(R_{14})(R_{15})]_n-C(R_{16})(R_{17})$- (IIIo), eine Gruppe der Formel $HN(R_{11}')-C(=O)-C(R_{16})(R_{17})$- (IIIp) oder eine Gruppe der Formel $HN(R_{11}')-C(=O)$- (IIIq) aufweisen, die Amidgruppe in den Gruppen IIIo bzw. IIIp bzw. IIIq zu der Gruppe $-CH_2-NH(R_{11}')$ (IIIr) reduzieren, wobei als Reduktionsmittel z. B. komplexe Metallhydride, wie $LiAlH_4$, Verwendung finden. Man erhält so Verbindungen III, die an dem die Gruppe $H(R_{11}')N$- (IIIj) tragenden Kohlenstoffatom zwei Wasserstoffatome aufweisen.

Ebenso kann man auch in üblicher Weise in Nitroalkylverbindungen der Formel IIIs die Nitroalkylgruppe zur Aminoalkylgruppe reduzieren, wobei die Verbindungen IIIs den Verbindungen III entsprechen, jedoch anstelle der in den Verbindungen III vorhandenen Gruppe IIIa eine Gruppe der Formel $O_2N-[C(R_{12})(R_{13})]_m-[C(R_{14})(R_{15})]_n-C(R_{16})(R_{17})$- (IIIt) aufweisen, und wobei als Reduktionsmittel z. B. komplexe Metallhydride, wie $LiAlH_4$, Verwendung finden. Man erhält so Verbindungen III, in denen $R_{11}'$ Wasserstoff ist.

Verbindungen III, in denen $R_{11}'$ Wasserstoff ist, können in üblicher Weise zu Verbindungen III, worin $R_{11}'$ $C_1$-$C_8$-Alkyl oder Benzyl ist, $C_1$-$C_8$-alkyliert oder benzyliert werden. Dazu setzt man z. B. die Verbindung III, worin $R_{11}'$ Wasserstoff ist, vorteilhaft in Gegenwart einer Base, z. B. in Gegenwart einer der vorstehend angegebenen Basen, und in einem inerten Lösungs- oder Verdünnungsmittel, z. B. der vorstehend angegebenen Art, mit einer Verbindung der Formel $C_1$-$C_8$-Alkyl-Z (IIIu) oder mit einer Verbindung der Formel Benzyl-Z (IIIv) um, wobei Z jeweils die vorstehend angegebene Bedeutung hat.

Die Verbindungen IIIb, IIIc, IIIk, IIIi, IIIs, IIIu und IIIv sind bekannt oder können in Analogie zu bekannten Verbindungen hergestellt werden.

Variante b):

Die N-Alkanoylierung, N-Benzoylierung, N-Alkylthiolierung, N-Phenylthiolierung oder N-Benzylthiolierung einer Verbindung I, worin $R_{11}$ Wasserstoff ist, oder gegebenenfalls eines Tautomeren davon, jeweils in freier Form oder in Salzform, erhältlich beispielsweise gemäss der Verfahrensvariante a), erfolgt in üblicher Weise, z. B. durch Umsetzung einer Verbindung I, worin $R_{11}$ Wasserstoff ist, oder gegebenenfalls eines Tautomeren davon, jeweils in freier Form oder in Salzform, vorteilhaft in Gegenwart einer Base, z. B. in Gegenwart einer der unter der Variante a) angegebenen Basen, in einem inerten Lösungs- oder Verdünnungsmittel, z. B.

in einem inerten Lösungs- oder Verdünnungsmittel der unter der Variante a) angegebenen Art, und in einem Temperaturbereich von etwa -80°C bis etwa +180°C, bevorzugt von etwa 0°C bis etwa +130°C, in vielen Fällen bei der Rückflusstemperatur des verwendeten Lösungsmittels, mit einer Verbindung der Formel $C_1$-$C_8$-Alkanoyl-Z (If), mit einer Verbindung der Formel Phenylcarbonyl-Z (Ig), deren Phenylgruppe unsubstituiert oder durch einen, zwei oder drei Substituenten, ausgewählt aus der Gruppe bestehend aus Halogen und $C_1$-$C_4$-Alkyl, substituiert ist, mit einer Verbindung der Formel $C_1$-$C_8$-Alkylthio-Z (Ih), mit einer Verbindung der Formel Halogen-$C_1$-$C_8$-alkylthio-Z (Ii), mit einer Verbindung der Formel Cyano-$C_1$-$C_8$-alkylthio-Z (Ij), mit einer Verbindung der Formel Phenylthio-Z (Ik) oder mit einer Verbindung der Formel Benzylthio-Z (Il), wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind.

In den Verbindungen If, Ig, A, Ii, Ij, Ik und Il hat Z jeweils die unter der Variante a) angegebene Bedeutung.

Die Verbindungen If, Ig, Ih, Ii, Ij, Ik und Il sind bekannt oder können in Analogie zu bekannten Verbindungen hergestellt werden.

Eine verfahrensgemäss oder auf andere Weise erhältliche Verbindung I oder gegebenenfalls ein Tautomeres davon kann in an sich bekannter Weise in eine andere Verbindung I überführt werden, indem man einen oder mehrere Substituenten der Ausgangsverbindung I in üblicher Weise durch (einen) andere(n) erfindungsgemässe(n) Substituenten ersetzt.

Beispielsweise können:

- nicht-halogenhaltige Substituenten bzw. nicht-halogenierte aromatische Ring-Teilstrukturen zu erfindungsgemässen halogenhaltigen Substituenten bzw. halogenierten aromatischen Ring-Teilstrukturen halogeniert werden;
- nicht-cyanohaltige Substituenten bzw. nicht-cyanosubstituierte aromatische Ring-Teilstrukturen in erfindungsgemässe cyanohaltige Substituenten bzw. cyanosubstituierte aromatische Ring-Teilstrukturen überführt werden;
- nicht-nitrierte aromatische Ring-Teilstrukturen zu erfindungsgemässen nitrierten aromatischen Ring-Teilstrukturen nitriert werden;
- nicht-(alkoxy-, alkylthio-, alkansulfinyl-, alkansulfonyl- oder halogen-)alkylierte aromatische Ring-Teilstrukturen zu erfindungsgemässen (alkoxy-, alkylthio-, alkansulfinyl-, alkansulfonyl- oder halogen-)alkylierten aromatischen Ring-Teilstrukturen (alkoxy-, alkylthio-, alkansulfinyl-, alkansulfonyl- oder halogen-) alkyliert werden;
- nicht-alkoxylierte Substituenten bzw. nicht-alkoxylierte aromatische Ring-Teilstrukturen zu erfindungsgemässen alkoxylierten Substituenten bzw. alkoxylierten aromatischen Ring-Teilstrukturen alkoxyliert werden;
- nicht-alkylthiolierte Substituenten bzw. nicht-alkylthiolierte aromatische Ring-Teilstrukturen zu erfindungsgemässen alkylthiolierten Substituenten bzw. alkylthiolierten aromatischen Ring-Teilstrukturen alkylthioliert werden;
- Alkylthio-Substituenten zu Alkylsulfinyl- oder Alkylsulfonyl-Substituenten oxidiert werden;
- Alkylsulfinyl-Substituenten zu Alkylsulfonyl-Substituenten oxidiert werden;
- nicht-phenylierte aromatische Ring-Teilstrukturen zu erfindungsgemässen phenylierten aromatischen Ring-Teilstrukturen phenyliert werden, wobei der einzuführende Phenyl-Substituent gegebenenfalls wie in der Definition der entsprechenden Variablen der Verbindungen I angegeben substituiert ist;
- nicht-phenoxylierte aromatische Ring-Teilstrukturen zu erfindungsgemässen phenoxylierten aromatischen Ring-Teilstrukturen phenoxyliert werden, wobei der Phenylring des einzuführenden Phenoxy-Substituenten gegebenenfalls wie in der Definition der entsprechenden Variablen der Verbindungen I angegeben substituiert ist;
- nicht-phenylthiolierte Substituenten bzw. nicht-phenylthiolierte aromatische Ring-Teilstrukturen zu erfindungsgemässen phenylthiolierten Substituenten bzw. phenylthiolierten aromatischen Ring-Teilstrukturen phenylthioliert werden, wobei der Phenylring des einzuführenden Phenylthio-Substituenten gegebenenfalls wie in der Definition der entsprechenden Variablen der Verbindungen I angegeben substituiert ist;
- nicht-alkanoylierte Substituenten bzw. nicht-alkanoylierte aromatische Ring-Teilstrukturen zu erfindungsgemässen alkanoylierten Substituenten bzw. alkanoylierten aromatischen Ring-Teilstrukturen alkanoyliert werden;
- nicht-alkansulfinylierte aromatische Ring-Teilstrukturen zu erfindungsgemässen alkansulfinylierten aromatischen Ring-Teilstrukturen alkansulfinyliert werden;
- nicht-alkansulfonylierte aromatische Ring-Teilstrukturen zu erfindungsgemässen alkansulfonylierten aromatischen Ring-Teilstrukturen alkansulfonyliert werden;
- nicht-phenylsulfonylierte aromatische Ring-Teilstrukturen zu erfindungsgemässen phenylsulfonylierten

aromatischen Ring-Teilstrukturen phenylsulfonyliert werden, wobei der Phenylring des einzuführenden Phenylsulfonyl-Substituenten gegebenenfalls wie in der Definition der entsprechenden Variablen der Verbindungen I angegeben substituiert ist;
- nicht-cycloalkylierte Substituenten bzw. nicht-cycloalkylierte aromatische Ring-Teilstrukturen zu erfindungsgemässen cycloalkylierten Substituenten bzw. cycloalkylierten aromatischen Ring-Teilstrukturen cycloalkyliert werden;
- nicht-alkenylierte aromatische Ring-Teilstrukturen zu erfindungsgemässen alkenylierten aromatischen Ring-Teilstrukturen alkenyliert werden;
- nicht-alkinylierte aromatische Ring-Teilstrukturen zu erfindungsgemässen alkinylierten aromatischen Ring-Teilstrukturen alkinyliert werden;
- nicht-hydroxylierte aromatische Ring-Teilstrukturen zu erfindungsgemässen hydroxylierten aromatischen Ring-Teilstrukturen hydroxyliert werden;
- nicht-aminosubstituierte aromatische Ring-Teilstrukturen zu erfindungsgemässen aminosubstituierten aromatischen Ring-Teilstrukturen aminosubstituiert werden, wobei Amino in "aminosubstituiert" für Amino, eine Gruppe Ic, eine Gruppe Id oder eine Gruppe Ie steht;
- nicht-alkylierte Substituenten zu erfindungsgemässen alkylierten Substituenten alkyliert werden;
- nicht-benzylierte Substituenten zu erfindungsgemässen benzylierten Substituenten benzyliert werden;
- nicht-phenylcarbonylierte Substituenten zu erfindungsgemässen phenylcarbonylierten Substituenten phenylcarbonyliert werden, wobei der Phenylring des einzuführenden Phenylcarbonyl-Substituenten gegebenenfalls wie in der Definition der entsprechenden Variablen der Verbindungen I angegeben substituiert ist;
- nicht-benzylthiolierte Substituenten zu erfindungsgemässen benzylthiolierten Substituenten benzylthioliert werden, wobei der Phenylring des einzuführenden Benzylthio-Substituenten gegebenenfalls wie in der Definition der entsprechenden Variablen der Verbindungen I angegeben substituiert ist;
- nicht-halogenalkylthiolierte Substituenten bzw. nicht-halogenalkylthiolierte aromatische Ring-Teilstrukturen zu erfindungsgemässen halogenalkylthiolierten Substituenten bzw. halogenalkylthiolierten aromatischen Ring-Teilstrukturen halogenalkylthioliert werden;
- nicht-halogenalkoxylierte aromatische Ring-Teilstrukturen zu erfindungsgemässen halogenalkoxylierten aromatischen Ring-Teilstrukturen halogenalkoxyliert werden;
- nicht-(halogen- oder cyano-)alkansulfonylierte aromatische Ring-Teilstrukturen zu erfindungsgemässen (halogen- oder cyano-)alkansulfonylierten aromatischen Ring-Teil-strukturen (halogen- oder cyano-)alkansulfonyliert werden;
- nicht-halogenalkenylierte aromatische Ring-Teilstrukturen zu erfindungsgemässen halogenalkenylierten aromatischen Ring-Teilstrukturen halogenalkenyliert werden;
- nicht-cyanoalkylthiolierte Substituenten zu erfindungsgemässen cyanoalkylthiolierten Substituenten cyanoalkylthioliert werden;
- nicht-(alkoxy- oder halogen-)alkylierte Substituenten zu erfindungsgemässen (alkoxy- oder halogen-)alkylierten Substituenten (alkoxy- oder halogen-)alkyliert werden;
- Halogensubstituenten in halogenierten aromatischen Ring-Teilstrukturen durch erfindungsgemässe Alkylthio-, Alkoxy-, Cyano-, Phenoxy- oder Phenylthio-Substituenten ersetzt werden, wobei der Phenylring des einzuführenden Phenoxy- oder Phenylthio-Substituenten gegebenenfalls wie in der Definition der entsprechenden Variablen der Verbindungen I angegeben substituiert ist.

Es ist dabei, je nach Wahl der dafür jeweils geeigneten Reaktionsbedingungen und Ausgangsmaterialien, möglich, in einem Reaktionsschritt nur einen Substituenten durch einen anderen erfindungsgemässen Substituenten zu ersetzen, oder es können in demselben Reaktionschritt mehrere Substituenten durch andere erfindungsgemässe Substituenten ersetzt werden. Beispielsweise können gleichzeitig zwei oder mehrere gleiche Halogensubstituenten in denselben oder, sofern vorhanden, in verschiedene Substituenten und/oder Ringe eingeführt werden. Ebenso kann z. B. ein bereits durch einen bestimmten Substituenten, z. B. Chlor, beispielsweise einfach, substituierter Substituent oder Ring - im Rahmen der erfindungsgemässen Definitionen der Variablen - durch einen oder mehrere weitere gleichartige Substituenten zusätzlich substituiert, z. B. chloriert, werden, d. h. beispielsweise in den di- oder einen noch höher-substituierten, z. B. -chlorierten, Zustand überführt werden.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Die Verbindungen I und gegebenenfalls ihre Tautomeren, jeweils in freier Form oder in Salzform, können teilweise in Form eines der möglichen Isomeren oder als Gemisch derselben, z. B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen I und gegebenenfalls ihrer Tautomeren, jeweils in freier Form oder in Salzform, können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird, oder durch Überführung in diastereomere Salze, z. B. durch Umsetzung eines basischen Endstoffracemats mit einer optisch aktiven Säure, wie Carbonsäure, z. B. Campher-, Wein- oder Äpfelsäure, oder Sulfonsäure, z. B. Camphersulfonsäure, und Trennung des auf diese Weise erhaltenen Diastereomerengemisches, z. B. auf Grund ihrer verschiedenen Löslichkeiten durch fraktionierte Kristallisation, in die Diastereomeren, aus denen das gewünschte Enantiomere durch Einwirkung geeigneter, z. B. basischer, Mittel freigesetzt werden kann.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfähren mit Edukten mit entsprechend geeigneter Stereochemie ausführt.

Es ist möglich, dass die biologische Wirksamkeit von Diastereomeren bzw. von Enantiomeren unterschiedlich ist. In diesem Fall isoliert bzw. synthetisiert man vorteilhaft jeweils das biologisch wirksamere Isomere, z.B. Enantiomere, oder Diastereomere.

Die Verbindungen I und gegebenenfalls ihre Tautomeren, jeweils in freier Form oder in Salzform, können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfähren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen.

Die Erfindung betrifft insbesondere die in den Beispielen H-1 bis H-14.16 beschriebenen Herstellungsverfahren.

Erfindungsgemäss für die Herstellung der Verbindungen I bzw. ihrer Salze verwendete Ausgangsstoffe und Zwischenprodukte, jeweils in freier Form oder in Salzform, die neu sind, ihre Verwendung und Verfahren zu ihrer Herstellung bilden ebenfalls einen Gegenstand der Erfindung.

Bevorzugte Zwischenprodukte sind solche der Formel IV,

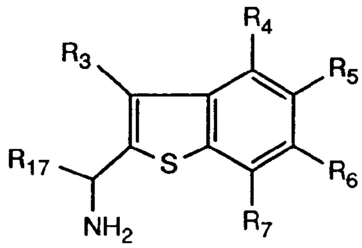

IV

worin

$R_3$, $R_4$, $R_5$, $R_6$ und $R_7$, unabhängig voneinander, Wasserstoff, Halogen, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, Halogen-$C_1$-$C_8$-alkoxy, $C_1$-$C_8$-Alkylthio, Halogen-$C_1$-$C_8$-alkylthio, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_4$-Alkyl und $C_1$-$C_4$-Alkoxy, substituiert sind, mit der Massgabe, dass von den fünf Resten $R_3$, $R_4$, $R_5$, $R_6$ und $R_7$, mindestens zwei Reste für Wasserstoff stehen und mindestens vier Reste von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind; und

$R_{17}$ Ethyl bedeuten;

in freier Form oder in Salzform.

Hiervon besonders bevorzugt sind solche, worin

$R_3$, $R_4$, $R_5$, $R_6$ und $R_7$ unabhängig voneinander Wasserstoff, Methyl, Ethyl, Methoxy oder Chlor bedeuten;

in freier Form oder in Salzform.

Namentlich bevorzugt sind:

(d,l)-2-(1-Aminopropyl)-benzo[b]thiophen;

(-)-2-(1-Aminopropyl)-benzo[b]thiophen,

in freier Form oder in Salzform.

In 4-Position aminosubstituierte Pyrimidine sind bereits bekannt, z. B. aus der europäischen Patentanmeldung mit der Veröffentlichungsnummer 0 126 254. Von diesen bekannten Verbindungen unterscheiden sich die Verbindungen I der vorliegenden Erfindung in charakeristischer Weise dadurch, dass in letzteren die Aminogruppe in 4-Position des Pyrimidinrings durch einen Benzo[b]furylalkyl-, Benzo[b]thienylalkyl- oder Indolylalkyl-Rest substituiert ist; zudem zeigen die Verbindungen I der vorliegenden Erfindung eine unerwartet hohe mikrobizide und akarizide Wirksamkeit.

Die Verbindungen I und gegebenenfalls ihre Tautomeren, jeweils in freier Form oder in Salzform, der vorliegenden Erfindung sind bei Raumtemperatur stabile Oele, Harze oder Feststoffe. Sie lassen sich auf dem Agrarsektor und verwandten Gebieten präventiv und/oder kurativ als Wirkstoffe bei der Bekämpfung von pflanzenschädigenden Mikroorganismen und von Schädlingen der Ordnung Akarina einsetzen. Die erfindungsgemässen Wirkstoffe der Formel I zeichnen sich - auch bei niedrigen Anwendungskonzentrationen - nicht nur durch hervorragende Wirkung, sondern auch durch gute Pflanzenverträglichkeit aus.

Die erfindungsgemässen Wirkstoffe der Formel I weisen ein für praktische Bedürfnisse sehr günstiges biozides Spektrum zur Bekämpfung von Schädlingen der Ordnung Akarina und von phytopathogenen Mikroorganismen, insbesondere Fungi, auf. Sie besitzen sehr vorteilhafte, insbesondere systemische, Eigenschaften und können zum Schutz von zahlreichen Kulturflanzen eingesetzt werden. Mit den Wirkstoffen der Formel I können an Pflanzen oder Pflanzenteilen (Früchten, Blüten, Laubwerk Stengeln, Knollen, Wurzeln) von unterschiedlichen Nutzkulturen die auftretenden Schädlinge eingedämmt oder vernichtet werden, wobei auch später zuwachsende Pflanzenteile, z. B. von phytopathogenen Mikroorganismen, verschont bleiben.

Verbindungen I sind z. B. gegen die den folgenden Klassen angehörenden phytopathogenen Pilze wirksam: Fungi imperfecti (z. B. Botrytis, Pyricularia, Helminthosporium, Fusarium, Septoria, Cercospora und Alternaria) und Basidiomyceten (z. B. Rhizoctonia, Hemileia, Puccinia). Darüber hinaus wirken sie gegen die Klassen der Ascomyceten (z. B Venturia und Erysiphe, Podosphaera Monilinia, Uncinula) und der Oomyceten (z. B. Phytophthora, Pythium, Plasmopara).

Die Verbindungen I können ferner als Beizmittel zur Behandlung von Saatgut (Früchten, Knollen, Körnern) und Pflanzenstecklingen zum Schutz vor Pilzinfektionen sowie gegen im Erdboden auftretende phytopathogene Pilze eingesetzt werden.

Die erfindungsgemässen Verbindungen I sind zudem wertvolle Wirkstoffe bei der Bekämpfung von Schädlingen der Ordnung Akarina an Nutz- und Zierpflanzen in der Landwirtschaft, insbesondere in Baumwoll-, Gemüse- und Obstpflanzungen, im Forst, im Vorrats- und Materialschutz-Bereich sowie im Hygienesektor, insbesondere an Haus- und Nutztieren. Sie sind gegen verschiedene Entwicklungsstadien wirksam. Dabei kann sich ihre Wirkung in unmittelbarer Abtötung der Schädlinge oder erst nach einiger Zeit, beispielsweise bei einer Häutung, oder in einer deutlich verminderten Eiablage und/oder Schlupfrate zeigen. Zur Ordnung Akarina gehören z. B. Boophilus spp. und Tetranychus spp.; diese Aufzählung ist nicht limitierend.

Die Erfindung betrifft auch die Mittel, die als Wirkstoff Verbindungen I enthalten, insbesondere pflanzenschützende Mittel, sowie deren Verwendung auf dem Agrarsektor und verwandten Gebieten. Darüber hinaus schliesst die Erfindung auch die Herstellung dieser Mittel ein, die gekennzeichiet ist durch das innige Vermischen der Aktivsubstanz mit einer oder mehreren der nachstehend beschriebenen Substanzen bzw. Substanzgruppen. Eingeschlossen in die Erdindung ist auch ein Verfahren zur Behandlung von Pflanzen, das sich durch Applikation der neuen Verbindungen I bzw. der neuen Mittel auszeichnet.

Als Zielkulturen für den pflanzenschützenden Einsatz gelten im Rahmen der Erfindung beispielsweise folgende Pflanzenarten: Getreide (Weizen, Gerste, Roggen, Hafer, Reis, Mais, Sorghum und verwandte Spezies); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Campfer) und Pflanzen wie Tabak, Nüsse, Kaffee, Eierfrüchte, Zuckerrohr, Tee, Pfeffer, Reben, Hopfen, Bananen- und Naturkautschuk-gewächse sowie Zierpflanzen und Rasen.

Wirkstoffe I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können z. B. Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden, ohne dass die Wirkung der Verbindungen der Formel I dadurch vermindert wird.

Geeignete Träger und Zusätze können fest oder flüssig sein und sind in der Formulierungstechnik zweckdienliche Stoffe, z. B. natürliche oder regenerierte mineralische Stoffe, Lösungs-, Dispergier-, Netz-, Haft-, Verdickungs-, Binde- oder Düngemittel.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe I können aber auch über den Erdboden durch das Wurzelwerk in der Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Bei Wasserreiskulturen kann man solche Granulate dem überfluteten Reisfeld zudosieren. Die Verbindungen I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen I werden dabei in unveränderter Form oder vorzugsweise zusammen mit den in der Formulierungstechnik üblichen Hilfsmitteln eingesetzt. Dafür werden sie zweckmässigerweise z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln oder Granulaten, z. B. durch Verkapselung in, z. B. polymeren, Stoffen, in bekannter Weise verarbeitet. Die Anwendungsverfahren wie Versprühen, Vernebeln, Verstäuben, Verstreuen, Bestreichen oder Giessen werden ebenso wie die Art der Mittel den angestrebten Zielen und den gegebenen Verhältnissen entsprechend gewählt.

Günstige Aufwandmengen liegen im allgemeinen bei 5 g bis 2 kg Aktivsubstanz (AS) je Hektar (ha), bevorzugt bei 10 g bis 1 kg AS/ha, insbesondere bei 20 g bis 600 g AS/ha; bei der Behandlung des Saatgutes bei 10 mg bis 1 g AS pro kg Saatgut.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen des Wirkstoffs mit Streckmitteln, wie Lösungsmitteln, festen Trägerstoffen und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glycole sowie deren Ether und Ester, wie Ethanol, Ethylenglycol, Ethylenglycolmonomethyl- oder -ethylether, Ketone, wie Cyclohexanon,

stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, und Wasser.

Als feste Trägerstoffe, z. B. für Stäubemittel und dispergierbare Pulver, werden in der Regel natürliche Gesteinsmehle verwendet, wie Calcit, Talkum , Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie Bimsstein, Ziegelbruch, Sepiolit oder Bentonit als nicht-sorptive Trägermaterialien z. B. Calcit oder Sand in Frage. Darüber hinaus kann eine Vierzahl von vorgranulierten Materialien anorganischer Natur, wie Dolomit oder zekleinerte Pflanzenrückstände, verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglycolether, Polypropylen-/Polyethylen-oxidaddukte, Tributylphenoxypolyethylenethanol, Polyethylenglycol und Octylphenoxypolyethoxyethanol erwähnt.

Ferner kommen auch Fettsäureester von Polyoxyethylensorbitan, wie das Polyoxyethylensorbitantrioleat, in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyle-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gewichtsprozent, insbesondere 0,1 bis 95 Gewichtsprozent, Wirkstoff der Formel I, 99,9 bis 1 Gewichtsprozent, insbesondere 99,8 bis 5 Gewichtsprozent, eines festen oder flüssigen Zusatzstoffes und 0 bis 25 Gewichtsprozent, insbesondere 0,1 bis 25 Gewichtsprozent, eines Tensids.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel oder Haftmittel, sowie Dünger oder andere Wirkstoffe zur Erzielung spezieller Effekte enthalten.

Die nachfolgenden Beispiele illustrieren die vorstehend beschriebene Erfindung, ohne dieselbe in ihrem Umfang in irgendeiner Weise einzuschränken. Temperaturen sind in Celsiusgraden angegeben. Die folgenden Abkürzungen werden verwendet: Ac = Acetyl; d = Dublett; dd = dublettiertes Dublett; Et = Ethyl; m = Multiplett; Me = Methyl; Ph = Phenyl; c-Pr = Cyclopropyl; Pr = n-Propyl; q = Quartett; s = Singulett; t = Triplett; Smp. = Schmelzpunkt. In den Beispielen F-1.1 bis F-6.3 steht der Ausdruck "%" für "Gewichtsprozent", ebenso im Rahmen von Konzentrationsangaben von Lösungen in den Beispielen B-1.1 bis B-11, sofern entsprechende Konzentrationen nicht in einer anderen Einheit angegeben sind. Unter dem Betriff "erfindungsgemässer Wirkstoff" ist in den Beispielen F-1.1 bis F-6.3 und B-1.1 bis B-11 jeweils eine Verbindung I oder gegebenenfalls ein Tautomeres davon, jeweils in freier Form oder in Form eines agrochemisch verwendbaren Salzes, zu verstehen, insbesondere eine solche Verbindung, gegebenenfalls in tautomerer Form, die als Produkt in den Beispielen H-1 bis H-9.6 beschrieben ist.

## Beispiele H-1 bis H-18: Herstellung erfindungsgemässer Verbindungen

### Beispiel H-1:

In einem Sulfierkolben wird ein Gemisch aus 1,86 g 4,5-Dichlor-6-ethylpyrimidin, 1,80 g 2-(1-Aminoethyl)-1-methyl-indol, 1,21 g Triethylamin und 25 g absolutem Isopropanol zum Rückfluss erhitzt, 12 Stunden bei einer Innentemperatur von 80 bis 82° gerührt und dann im Wasserstrahlvakuum vom Lösungsmittel befreit. Der Rückstand wird in einem Gemisch aus 250 ml Ethylacetat und 80 ml Wasser aufgenommen und die organische Phase zweimal mit Wasser extrahiert, über Natriumsulfat getrocknet und eingedampft. Der Rückstand wird mittels Flash-Chromatographie an Kieselgel mit Ethylacetat/Hexan (1:3) als Laufmittel gereinigt. Man erhält so das 2-[1-(5-Chlor-6-ethylpyrimidin-4-ylamino)ethyl]-1-methyl-indol, das bei 129 bis 131° schmilzt.

Beispiel H-2:

In analoger Weise wie in Beispiel H-1 beschrieben oder mittels einer entsprechenden anderen der vorstehend angegebenen Verfahrensweisen können auch die in der nachfolgenden Tabelle 1 aufgeführten Verbindungen der allgemeinen Formel I.1 hergestellt werden.

Tabelle 1:

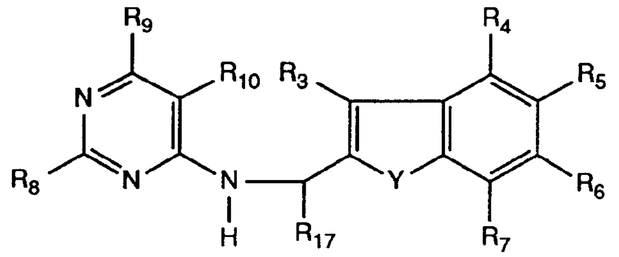

(I.1)

| Bsp. | $R_3$ | $R_4$ | $R_5$ | $R_6$ | $R_7$ | $R_8$ | $R_9$ | $R_{10}$ | $R_{17}$ | Y | phys. Daten |
|---|---|---|---|---|---|---|---|---|---|---|---|
| H-2.1 | H | H | H | H | H | H | Me | Cl | Me | N(Me) | |
| H-2.2 | H | H | H | H | H | H | Et | Br | Me | N(Me) | |
| H-2.3 | H | H | H | H | H | H | Me | Br | Me | N(Me) | |
| H-2.4 | H | H | H | H | H | H | Cl | Et | Me | N(Me) | |
| H-2.5 | Me | H | H | H | H | H | Et | Cl | Me | N(H) | Smp. 117-119°C |
| H-2.6 | Me | H | H | H | H | H | Me | Cl | Me | N(H) | |
| H-2.7 | Me | H | H | H | H | H | Et | Cl | Me | N(Ac) | |
| H-2.8 | Me | H | H | H | H | H | Me | Cl | Me | N(Ac) | |
| H-2.9 | Me | H | H | H | H | H | Et | Cl | Me | N(Me) | |
| H-2.10 | Me | H | H | H | H | H | Me | Cl | Me | N(Me) | |
| H-2.11 | Me | H | H | H | H | H | Et | Br | Me | N(Me) | |
| H-2.12 | H | H | H | H | H | H | F | Et | Me | N(Me) | |
| H-2.13 | H | H | H | H | H | H | Et | Cl | Me | S | Smp. 82-83°C |
| H-2.14 | H | H | H | H | H | Me | Et | Cl | Me | S | |
| H-2.15 | H | H | H | H | H | H | Et | Cl | Et | S | Oel, $^1$H-NMR |
| H-2.16 | H | H | H | H | H | H | Et | Br | Me | S | |
| H-2.17 | H | H | H | H | H | H | Et | $NO_2$ | Me | S | |
| H-2.18 | Me | H | H | H | H | H | Et | Cl | Me | S | Smp. 50-51°C |
| H-2.19 | Me | H | H | H | H | H | Et | Cl | Et | S | Smp. 77-78°C |
| H-2.20 | H | H | Cl | H | H | H | Et | Cl | Me | S | |
| H-2.21 | H | H | Cl | H | H | H | Me | Cl | Me | S | |
| H-2.22 | H | H | H | H | H | H | Et | Cl | Me | O | |
| H-2.23 | H | H | H | H | H | H | Me | Cl | Et | O | |
| H-2.24 | H | H | H | H | H | H | Et | Br | Me | O | |
| H-2.25 | H | H | Cl | H | H | H | Me | Cl | Me | O | |

| Bsp. | $R_3$ | $R_4$ | $R_5$ | $R_6$ | $R_7$ | $R_8$ | $R_9$ | $R_{10}$ | $R_{17}$ | Y | phys. Daten |
|---|---|---|---|---|---|---|---|---|---|---|---|
| H-2.26 | H | H | Cl | H | H | H | Et | Cl | Me | O | Smp. 135-136°C |
| H-2.27 | H | H | Cl | H | H | H | Et | Cl | Et | O | Smp. 67-69°C |
| H-2.28 | H | H | Cl | H | H | H | c-Pr | Cl | Me | O | |
| H-2.29 | H | H | OMe | H | H | H | Et | Cl | Me | O | Smp. 67-68°C |
| H-2.30 | H | H | OMe | H | H | H | Et | Cl | Et | O | Smp. 99-101°C |
| H-2.31 | H | H | OMe | H | H | H | Me | $NO_2$ | Me | O | |
| H-2.32 | H | H | OMe | H | H | H | Me | I | Me | O | |
| H-2.33 | H | H | H | H | OMe | H | Et | Cl | Me | O | Smp. 105-106°C |
| H-2.34 | H | H | H | H | OMe | H | Et | Br | Me | O | |
| H-2.35 | H | H | H | H | OMe | H | Me | Br | Me | O | |
| H-2.36 | H | H | H | H | OMe | H | Br | Et | Et | O | |
| H-2.37 | H | H | H | OMe | H | H | Et | Cl | Me | O | Smp. 106-107°C |
| H-2.38 | H | H | H | OMe | H | H | Et | Cl | Et | O | |
| H-2.39 | H | OMe | H | OMe | H | H | Et | Cl | Me | O | Smp. 87-88°C |
| H-2.40 | H | OMe | H | OMe | H | H | Et | Cl | Et | O | |
| H-2.41 | H | OMe | H | OMe | H | H | Me | Cl | Me | O | |
| H-2.42 | H | OMe | H | OMe | H | H | Et | F | Et | O | |
| H-2.43 | Me | H | H | H | H | H | Et | Cl | Me | O | Smp. 91-92°C |
| H-2.44 | Me | H | H | H | H | H | Et | Cl | Et | O | |
| H-2.45 | Me | H | H | H | H | H | Me | Cl | Me | O | |
| H-2.46 | Et | H | H | H | H | H | Et | Cl | Me | O | |
| H-2.47 | Et | H | H | H | H | H | Et | Cl | Et | O | |
| H-2.48 | Me | H | Cl | H | H | H | Et | Cl | Me | O | |
| H-2.49 | Me | H | Cl | H | H | H | Et | Cl | Et | O | |
| H-2.50 | Me | H | Cl | H | H | H | Me | Cl | Me | O | |
| H-2.51 | Me | H | Cl | H | H | H | Me | Br | Me | O | |
| H-2.52 | Me | H | Cl | H | H | H | Et | $NO_2$ | Me | O | |
| H-2.53 | Me | H | OMe | H | H | H | Et | Cl | Me | O | |
| H-2.54 | Me | H | OMe | H | H | H | Et | Cl | Et | O | |
| H-2.55 | Me | H | OMe | H | H | H | Me | Cl | Me | O | |
| H-2.56 | Me | H | OMe | H | H | H | Et | Br | Me | O | |
| H-2.57 | Me | H | OMe | H | H | Me | Cl | Me | Me | O | |
| H-2.58 | Me | H | OMe | H | H | Me | Cl | Et | Me | O | |

| Bsp. | $R_3$ | $R_4$ | $R_5$ | $R_6$ | $R_7$ | $R_8$ | $R_9$ | $R_{10}$ | $R_{17}$ | Y | phys. Daten |
|---|---|---|---|---|---|---|---|---|---|---|---|
| H-2.59 | H | H | Cl | H | H | H | Et | Cl | Et | S | |
| H-2.60 | H | H | Cl | H | H | H | Me | Cl | Et | S | |
| H-2.61 | Me | H | H | H | H | H | Et | Br | Et | S | Smp. 62-63°C |
| H-2.62 | Me | H | Cl | H | H | H | Et | Cl | Et | S | Oel, $^1$H-NMR |
| H-2.63 | Me | H | Cl | H | H | H | Et | Cl | Me | S | Smp. 91-92°C |
| H-2.64 | H | H | H | H | H | H | Et | Br | Et | S | |
| H-2.65 | H | H | H | H | H | H | Et | Cl | Et | NH | |
| H-2.66 | H | H | H | H | H | H | Et | Br | Et | NH | |
| H-2.67 | H | H | H | H | H | H | Me | Cl | Et | NH | |
| H-2.68 | H | H | H | H | H | H | Et | Cl | Me | NH | |
| H-2.69 | H | H | H | H | H | H | Me | Cl | Me | NH | |
| H-2.70 | H | H | H | H | H | H | Et | Br | Me | S | |
| H-2.71 | H | H | H | H | H | H | Me | Cl | Et | S | |
| H-2.72 | H | H | H | H | H | H | Me | Br | Et | S | |
| H-2.73 | H | H | H | H | H | H | Et | Cl | Me | N-$CH_2$Ph | |
| H-2.74 | H | H | H | H | H | H | Et | Cl | Et | N-$CH_2$Ph | |
| H-2.75 | H | H | H | H | H | H | Me | Cl | Me | N-$CH_2$Ph | |
| H-2.76 | H | H | H | H | H | H | Me | Cl | Et | N-$CH_2$Ph | |

Beispiele H-3.1:

In analoger Weise wie in Beispiel H-1 beschrieben oder mittels einer entsprechenden anderen der vorstehend angegebenen Verfahrensweisen können auch die in der nachfolgenden Tabelle 2 aufgeführten Verbindungen der allgemeinen Formel I.2 hergestellt werden.

Tabelle 2:

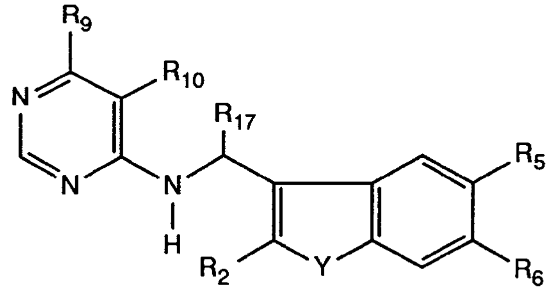

(I.2)

| Bsp. | $R_2$ | $R_5$ | $R_6$ | $R_9$ | $R_{10}$ | $R_{17}$ | Y | physikalische Daten |
|---|---|---|---|---|---|---|---|---|
| H-3.1 | H | H | H | Et | Cl | Me | N(H) | Smp.: 162-164° |
| H-3.2 | H | H | H | Et | Cl | Et | N(H) | |
| H-3.3 | H | H | H | Me | Br | Me | N(H) | |
| H-3.4 | H | H | H | Me | Cl | Me | N(H) | |
| H-3.5 | H | H | H | Et | Cl | Me | N(Ac) | |
| H-3.6 | H | H | H | Et | Cl | Et | N(Ac) | |
| H-3.7 | H | H | H | Me | Cl | Me | N(Ac) | |
| H-3.8 | H | H | H | Et | Cl | Me | $N(SO_2Ph)$ | |
| H-3.9 | H | H | H | Me | Cl | Me | $N(SO_2Ph)$ | |
| H-3.10 | Me | H | H | Et | Cl | Me | N(Me) | |
| H-3.11 | Me | H | H | Et | Cl | Et | N(Me) | |
| H-3.12 | Me | H | H | Me | Cl | Me | N(Me) | |
| H-3.13 | H | H | H | Et | Cl | Me | N(Me) | Brechungsindex: $n_D^{50} = 1.6022$ |
| H-3.14 | H | H | H | Me | Cl | Me | N(Me) | |
| H-3.15 | H | H | H | Me | Br | Me | N(Me) | |
| H-3.16 | H | H | H | Cl | Et | Me | N(Me) | |
| H-3.17 | H | H | H | Et | Cl | Me | S | Smp. 138-140°C |
| H-3.18 | H | H | H | Et | Cl | Et | S | |
| H-3.19 | H | H | H | Me | Cl | Me | S | |
| H-3.20 | Me | H | H | Et | Cl | Me | S | |
| H-3.21 | Me | H | H | Et | Cl | Et | S | |
| H-3.22 | Me | H | H | Me | Cl | Me | S | |
| H-3.23 | Me | H | H | Me | Br | Me | S | |
| H-3.24 | Me | H | H | Pr | Cl | Me | S | |

| Bsp. | $R_2$ | $R_5$ | $R_6$ | $R_9$ | $R_{10}$ | $R_{17}$ | Y | physikalische Daten |
|---|---|---|---|---|---|---|---|---|
| H-3.25 | Ph | H | H | Et | Cl | Me | S | |
| H-3.26 | Ph | H | H | Et | Cl | Et | S | |
| H-3.27 | Me | Cl | H | Et | Cl | Me | S | |
| H-3.28 | Me | Cl | H | Et | Cl | Et | S | |
| H-3.29 | Me | Cl | H | Me | Cl | Me | S | |
| H-3.30 | Me | Cl | H | Me | Br | Et | S | |
| H-3.31 | H | H | H | Et | Cl | Me | O | |
| H-3.32 | H | H | H | Et | Cl | Et | O | |
| H-3.33 | H | H | H | Me | Cl | Me | O | |
| H-3.34 | H | H | H | Me | Br | Me | O | |
| H-3.35 | H | H | H | Cl | Et | Et | O | |
| H-3.36 | Me | H | H | Et | Cl | Me | O | |
| H-3.37 | Me | H | H | Et | Cl | Et | O | |
| H-3.38 | Me | H | H | Me | Cl | Me | O | |
| H-3.39 | Me | H | H | Et | Br | Et | O | |
| H-3.40 | Ph | H | H | Et | Cl | Me | O | |
| H-3.41 | Ph | H | H | Et | Cl | Et | O | |
| H-3.42 | Me | Cl | H | Et | Cl | Me | O | |
| H-3.43 | Me | Cl | H | Et | Cl | Et | O | |
| H-3.44 | Me | Cl | H | Me | Br | Me | O | |
| H-3.45 | Me | Cl | H | Me | I | Et | O | |
| H-3.46 | Me | Cl | H | Pr | Cl | Me | O | |
| H-3.47 | Me | OMe | H | Et | Cl | Me | O | |
| H-3.48 | Me | OMe | H | Et | Cl | Et | O | |
| H-3.49 | Me | OMe | H | Me | Cl | Me | O | |
| H-3.50 | Me | OMe | H | Me | Br | Me | O | |
| H-3.51 | Me | H | F | Et | Cl | Me | O | |
| H-3.52 | Me | H | F | Et | Cl | Et | O | |
| H-3.53 | Me | H | F | Me | Cl | Me | O | |
| H-3.54 | Me | H | F | Me | Br | Me | O | |
| H-3.55 | Me | H | F | c-Pr | Cl | Me | O | |

Beispiele H-4:

In analoger Weise wie in Beispiel H-1 beschrieben oder mittels einer entsprechenden anderen der vorstehend angegebenen Verfahrensweisen können auch die in der nachfolgenden Tabelle 3 aufgeführten Verbindungen der allgemeinen Formel I.3 hergestellt werden.

Tabelle 3:

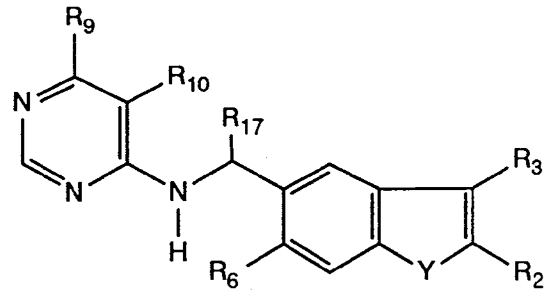

(I.3)

| Beispiel | $R_2$ | $R_3$ | $R_6$ | $R_9$ | $R_{10}$ | $R_{17}$ | Y | physikalische Daten |
|---|---|---|---|---|---|---|---|---|
| H-4.1 | H | H | Me | Et | Cl | Me | S | |
| H-4.2 | H | H | Me | Et | Cl | Et | S | |
| H-4.3 | H | H | Me | Me | Cl | Me | S | |
| H-4.4 | H | H | H | Et | Cl | Me | O | |
| H-4.5 | H | H | H | Et | Cl | Et | O | |
| H-4.6 | H | H | H | Me | Br | Me | O | |
| H-4.7 | Me | Me | Me | Et | Cl | Me | O | |
| H-4.8 | Me | Me | Me | Et | Cl | Et | O | |
| H-4.9 | Me | Me | Me | Me | Cl | Me | O | |
| H-4.10 | Me | Me | Me | Me | Br | Me | O | |
| H-4.11 | H | H | H | Et | Cl | Me | N(H) | |
| H-4.12 | H | H | H | Et | Cl | Et | N(H) | |
| H-4.13 | H | H | H | Et | Cl | Me | N(Ac) | |
| H-4.14 | H | H | H | Et | Cl | Et | N(Ac) | |
| H-4.15 | Ph | H | Me | Et | Cl | Me | O | |
| H-4.16 | Ph | H | Me | Et | Cl | Et | O | |
| H-4.17 | Ph | H | Me | Me | Cl | Me | O | |
| H-4.18 | Ph | H | Me | Me | Br | Me | O | |
| H-4.19 | Me | Me | Me | Et | Cl | Me | N(H) | |
| H-4.20 | Me | Me | Me | Et | Cl | Et | N(H) | |

| Beispiel | $R_2$ | $R_3$ | $R_6$ | $R_9$ | $R_{10}$ | $R_{17}$ | Y | physikalische Daten |
|---|---|---|---|---|---|---|---|---|
| H-4.21 | Me | Me | Me | Me | Cl | Me | N(H) | |
| H-4.22 | Me | Me | Me | Me | Br | Me | N(H) | |
| H-4.23 | Me | Me | Me | Et | Cl | Me | N(Ac) | |
| H-4.24 | Me | Me | Me | Et | Cl | Et | N(Ac) | |
| H-4.25 | Me | Me | Me | Me | Cl | Me | N(Ac) | |
| H-4.26 | Me | Me | Me | Me | Br | Me | N(Ac) | |

Beispiele H-5:

In analoger Weise wie in Beispiel H-1 beschrieben oder mittels einer entsprechenden anderen der vorstehend angegebenen Verfahrensweisen können auch die in der nachfolgenden Tabelle 4 aufgeführten Verbindungen der allgemeinen Formel I.4 hergestellt werden.

Tabelle 4:

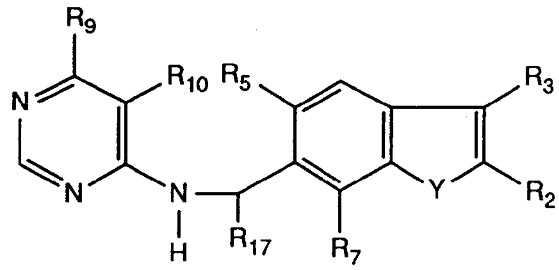

(I.4)

| Beispiel | $R_2$ | $R_3$ | $R_5$ | $R_7$ | $R_9$ | $R_{10}$ | $R_{17}$ | Y | physikalische Daten |
|---|---|---|---|---|---|---|---|---|---|
| H-5.1 | H | H | H | H | Et | Cl | Me | S | |
| H-5.2 | H | H | H | H | Et | Cl | Et | S | |
| H-5.3 | H | H | H | H | Me | Br | Me | S | |
| H-5.4 | H | H | H | H | Me | Cl | Me | S | |
| H-5.5 | H | H | H | H | Cl | Et | Me | S | |
| H-5.6 | Me | Me | H | H | Et | Cl | Me | O | Smp.: 79-80° |
| H-5.7 | Me | Me | H | H | Et | Cl | Et | O | Oel; $^1$H-NMR |
| H-5.8 | Me | Me | H | H | Me | Cl | Me | O | |
| H-5.9 | Me | Me | H | H | Me | Br | Me | O | |
| H-5.10 | H | H | H | Me | Et | Cl | Me | O | |
| H-5.11 | H | H | H | Me | Et | Cl | Et | O | |
| H-5.12 | H | H | H | Me | Me | Cl | Me | O | |
| H-5.13 | H | H | H | Br | Et | Cl | Me | O | |
| H-5.14 | H | H | H | Br | Et | Cl | Et | O | |
| H-5.15 | H | H | H | Br | Me | Br | Me | O | |
| H-5.16 | H | H | H | Br | Pr | Cl | Me | O | |
| H-5.17 | H | H | Me | H | Et | Cl | Me | O | |
| H-5.18 | H | H | Me | H | Et | Cl | Et | O | |
| H-5.19 | H | H | Me | H | Me | Cl | Me | O | |
| H-5.20 | H | H | Me | H | Me | Br | Me | O | |
| H-5.21 | H | H | Me | H | Me | F | Me | O | |
| H-5.22 | Me | Me | H | H | Et | Cl | Me | N(H) | Smp.: 128-130° |
| H-5.23 | Me | Me | H | H | Et | Cl | Et | N(H) | |
| H-5.24 | Me | Me | H | H | Me | Cl | Me | N(H) | |
| H-5.25 | Me | Me | H | H | Me | Br | Me | N(H) | |

| Beispiel | $R_2$ | $R_3$ | $R_5$ | $R_7$ | $R_9$ | $R_{10}$ | $R_{17}$ | Y | physikalische Daten |
|---|---|---|---|---|---|---|---|---|---|
| H-5.26 | Me | Me | H | H | Et | Cl | Me | N(Ac) | |
| H-5.27 | Me | Me | H | H | Et | Cl | Et | N(Ac) | |
| H-5.28 | Me | Me | H | H | Me | Cl | Me | N(Ac) | |
| H-5.29 | Me | Me | H | H | Me | Br | Me | N(Ac) | |
| H-5.30 | Me | Me | H | H | Et | Cl | Me | N(Me) | |
| H-5.31 | Me | Me | H | H | Et | Cl | Et | N(Me) | |
| H-5.32 | Me | Me | H | H | Me | Cl | Me | N(Me) | |
| H-5.33 | Me | Me | H | H | Me | Br | Me | N(Me) | |
| H-5.34 | Me | Me | H | H | Et | Cl | Me | S | Smp. 110-111°C |

Beispiele H-6:

In analoger Weise wie in Beispiel H-1 beschrieben oder mittels einer entsprechenden anderen der vorstehend angegebenen Verfahrensweisen können auch die in der nachfolgenden Tabelle 5 aufgeführten Verbindungen der allgemeinen Formel I.5 hergestellt werden.

Tabelle 5:

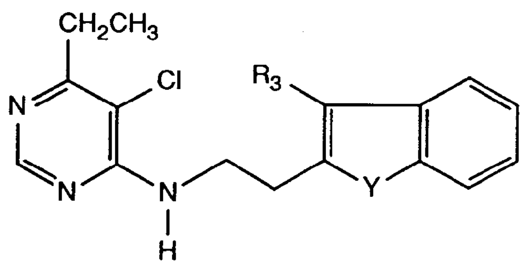

(I.5)

| Beispiel | $R_3$ | Y | physikalische Daten |
|---|---|---|---|
| H-6.1 | H | S | |
| H-6.2 | H | O | |
| H-6.3 | H | N(H) | |
| H-6.4 | Me | N(Me) | |

Beispiele H-7:

In analoger Weise wie in Beispiel H-1 beschrieben oder mittels einer entsprechenden anderen der vorstehend angegebenen Verfahrensweisen können auch die in der nachfolgenden Tabelle 6 aufgeführten Verbindungen der allgemeinen Formel I.6 hergestellt werden.

Tabelle 6:

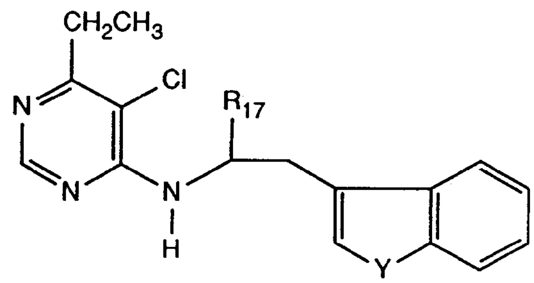

(I.6)

| Beispiel | $R_{17}$ | Y | physikalische Daten |
|---|---|---|---|
| H-7.1 | H | S | |
| H-7.2 | H | O | |
| H-7.3 | H | N(Me) | |
| H-7.4 | Me | S | |
| H-7.5 | Me | O | |
| H-7.6 | Me | N(Me) | |
| H-7.7 | Me | NH | |

Beispiele H-8:

In analoger Weise wie in Beispiel H-1 beschrieben oder mittels einer entsprechenden anderen der vorstehend angegebenen Verfahrensweisen können auch die in der nachfolgenden Tabelle 7 aufgeführten Verbindungen der allgemeinen Formel I.7 hergestellt werden.

Tabelle 7:

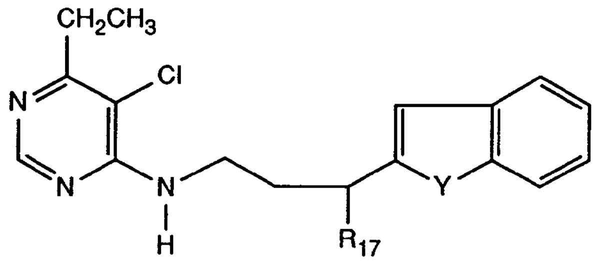

(I.7)

| Beispiel | $R_{17}$ | Y | physikalische Daten |
|---|---|---|---|
| H-8.1 | Me | S | |
| H-8.2 | H | S | |
| H-8.3 | Me | O | |
| H-8.4 | H | O | |
| H-8.5 | Me | N(Me) | |
| H-8.6 | H | N(Me) | |

Beispiele H-9:

In analoger Weise wie in Beispiel H-1 beschrieben oder mittels einer entsprechenden anderen der vorstehend angegebenen Verfahrensweisen können auch die in der nachfolgenden Tabelle 8 aufgeführten Verbindungen der allgemeinen Formel I.8 hergestellt werden.

Tabelle 8:

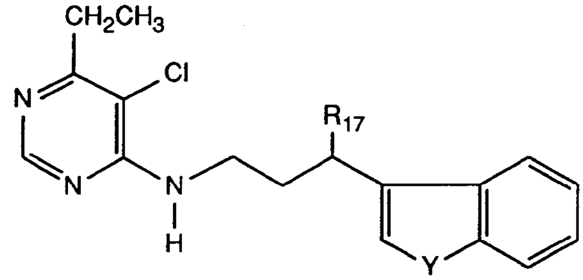

(I.8)

| Beispiel | $R_{17}$ | Y | physikalische Daten |
|---|---|---|---|
| H-9.1 | Me | S | |
| H-9.2 | H | S | |
| H-9.3 | Me | O | |
| H-9.4 | H | O | |
| H-9.5 | Me | N(Me) | |
| H-9.6 | H | N(Me) | |

Beispiel H-10:

Das in dem Verfahren von Beispiel H-2.18 verwendete Ausgangsmaterial kann z. B. folgendermassen hergestellt werden:
In einem Autoklav werden zu einem Gemisch aus 23,5 g 2-(1-Hydroxyiminoethyl)-3-methyl-benzo[b]thiophen und 250 ml absolutem Methanol 20 g trockenes Ammoniak und 23,5 g Raney-Nickel zugegeben. Dann wird das Reaktionsgemisch auf 50° erwärmt, bei dieser Temperatur ungefähr 5 Stunden unter einem Druck von ungefähr 100 bar hydriert und sodann auf Raumtemperatur abgekühlt, der Katalysator abfiltriert und das Filtrat im Wasserstrahlvakuum eingedampft. Der ölige Rückstand, das 2-( 1-Aminoethyl)-3-methyl-benzo[b]thiophen ($^1$H-NMR), wird ohne zusätzliche Reinigung weiterumgesetzt.

Beispiele H-11:

In analoger Weise wie in Beispiel H-10 beschrieben oder mittels einer entprechenden anderen der vorstehend angegebenen Verfahrensweisen können auch die in der nachfolgenden Tabelle 9 aufgeführten Verbindungen der allgemeinen Formel Ia.9 hergestellt werden, die z. B. als Ausgangsmaterialien in den Verfahren der Beispiele H-1 und H-2 dienen.

Tabelle 9:

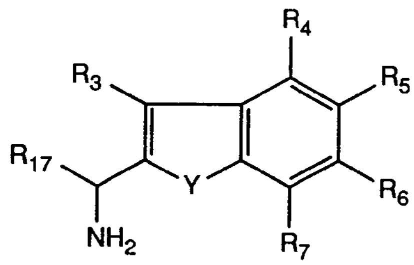

(Ia.9)

| Beispiel | $R_3$ | $R_4$ | $R_5$ | $R_6$ | $R_7$ | $R_{17}$ | Y | physikalische Daten |
|---|---|---|---|---|---|---|---|---|
| H-11.1 | H | H | H | H | H | Me | S | Oel; $^1$H-NMR |
| H-11.2 | H | H | H | H | H | Et | S | Oel; $^1$H-NMR |
| H-11.3 | Me | H | H | H | H | Et | S | Oel; $^1$H-NMR |
| H-11.4 | H | H | Cl | H | H | Me | S | |
| H-11.5 | H | H | Cl | H | H | Et | S | |
| H-11.6 | H | H | H | H | H | Me | O | |
| H-11.7 | H | H | H | H | H | Et | O | |
| H-11.8 | H | H | Cl | H | H | Me | O | Oel; $^1$H-NMR |
| H-11.9 | H | H | Cl | H | H | Et | O | Oel; $^1$H-NMR |
| H-11.10 | H | H | OMe | H | H | Me | O | Smp.: 40-41° |
| H-11.11 | H | H | OMe | H | H | Et | O | Oel; $^1$H-NMR |
| H-11.12 | H | H | H | H | OMe | Me | O | Oel; $^1$H-NMR |
| H-11.13 | H | H | H | H | OMe | Et | O | |
| H-11.14 | H | H | H | OMe | H | Me | O | Oel; $^1$H-NMR |
| H-11.15 | H | H | H | OMe | H | Et | O | |
| H-11.16 | H | OMe | H | OMe | H | Me | O | Oel; $^1$H-NMR |
| H-11.17 | H | OMe | H | OMe | H | Et | O | |
| H-11.18 | Me | H | H | H | H | Me | O | Oel; $^1$H-NMR |
| H-11.19 | Me | H | H | H | H | Et | O | |
| H-11.20 | Et | H | H | H | H | Me | O | |
| H-11.21 | Et | H | H | H | H | Et | O | |

| Beispiel | $R_3$ | $R_4$ | $R_5$ | $R_6$ | $R_7$ | $R_{17}$ | Y | physikalische Daten |
|---|---|---|---|---|---|---|---|---|
| H-11.22 | Me | H | Cl | H | H | Me | O | |
| H-11.23 | Me | H | Cl | H | H | Et | O | |
| H-11.24 | Me | H | OMe | H | H | Me | O | |
| H-11.25 | Me | H | OMe | H | H | Et | O | |
| H-11.26 | Me | H | H | H | H | Me | N(H) | Smp.: 92-95° |
| H-11.27 | Me | H | H | H | H | Me | N(Ac) | |
| H-11.28 | H | H | H | H | H | Me | N(Me) | Oel; $^1$H-NMR |
| H-11.29 | Me | H | H | H | H | Me | N(Me) | Oel |
| H-11.30 | Me | H | Cl | H | H | Me | S | Oel; $^1$H-NMR |
| H-11.31 | Me | H | Cl | H | H | Et | S | Oel; $^1$H-NMR |
| H-11.32 | Me | H | H | H | H | Me | S | Oel; $^1$H-NMR |
| H-11.33 | H | H | H | H | H | Me | N-$CH_2$Ph | |
| H-11.34 | H | H | H | H | H | Et | N-$CH_2$Ph | |

Beispiele H-12:

In analoger Weise wie in Beispiel H-10 beschrieben oder mittels einer entsprechenden anderen der vorstehend angegebenen Verfahrensweisen können auch die in der nachfolgenden Tabelle 10 aufgeführten Verbindungen der allgemeinen Formel Ia.10 hergestellt werden, die z. B. als Aüsgangsmaterialien in den Verfahren der Beispiele H-3 dienen.

Tabelle 10:

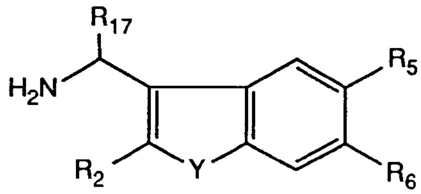

(Ia.10)

| Beispiel | $R_2$ | $R_5$ | $R_6$ | $R_{17}$ | Y | physikalische Daten |
|---|---|---|---|---|---|---|
| H-12.1 | H | H | H | Me | S | |
| H-12.2 | H | H | H | Et | S | |
| H-12.3 | Me | H | H | Me | S | |
| H-12.4 | Me | H | H | Et | S | |
| H-12.5 | Ph | H | H | Me | S | |
| H-12.6 | Ph | H | H | Et | S | |
| H-12.7 | Me | Cl | H | Me | S | |
| H-12.8 | Me | Cl | H | Et | S | |
| H-12.9 | H | H | H | Me | O | |
| H-12.10 | H | H | H | Et | O | |
| H-12.11 | Me | H | H | Me | O | |
| H-12.12 | Me | H | H | Et | O | |
| H-12.13 | Ph | H | H | Me | O | |
| H-12.14 | Ph | H | H | Et | O | |
| H-12.15 | Me | Cl | H | Me | O | |
| H-12.16 | Me | Cl | H | Et | O | |
| H-12.17 | Me | OMe | H | Me | O | |
| H-12.18 | Me | OMe | H | Et | O | |
| H-12.19 | Me | H | F | Me | O | |
| H-12.20 | Me | H | F | Et | O | |
| H-12.21 | H | H | H | Me | N(H) | Oel; $^1$H-NMR |
| H-12.22 | H | H | H | Et | N(H) | |
| H-12.23 | H | H | H | Me | N(Ac) | |
| H-12.24 | H | H | H | Et | N(Ac) | |
| H-12.25 | H | H | H | Me | N($SO_2$Ph) | |
| H-12.26 | H | H | H | Me | N(Me) | Oel; $^1$H-NMR |
| H-12.27 | Me | H | H | Me | N(Me) | |
| H-12.28 | Me | H | H | Et | N(Me) | |

Beispiele H- 13:

In analoger Weise wie in Beispiel H-10 beschrieben oder mittels einer entsprechenden anderen der vorstehend angegebenen Verfahrensweisen können auch die in der nachfolgenden Tabelle 11 aufgeführten Verbindungen der allgemeinen Formel Ia.11 hergestellt werden, die z. B. als Ausgangsmaterialien in den Verfahren der Beispiele H-4 dienen.

Tabelle 11:

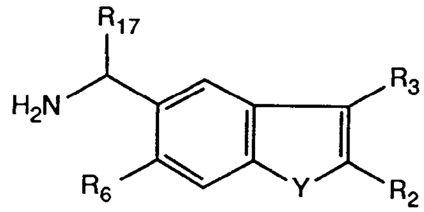

(Ia.11)

| Beispiel | $R_2$ | $R_3$ | $R_6$ | $R_{17}$ | Y | physikalische Daten |
|---|---|---|---|---|---|---|
| H-13.1 | H | H | Me | Me | S | |
| H-13.2 | H | H | Me | Et | S | |
| H-13.3 | H | H | H | Me | O | |
| H-13.4 | H | H | H | Et | O | |
| H-13.5 | Me | Me | Me | Me | O | |
| H-13.6 | Me | Me | Me | Et | O | |
| H-13.7 | Ph | H | Me | Me | O | |
| H-13.8 | Ph | H | Me | Et | O | |
| H-13.9 | Me | Me | Me | Me | N(H) | |
| H-13.10 | Me | Me | Me | Et | N(H) | |
| H-13.11 | Me | Me | Me | Me | N(Ac) | |
| H-13.12 | Me | Me | Me | Et | N(Ac) | |
| H-13.13 | H | H | H | Me | N(H) | |
| H-13.14 | H | H | H | Et | N(H) | |
| H-13.15 | H | H | H | Me | N(Ac) | |
| H-13.16 | H | H | H | Et | N(Ac) | |

Beispiele H-14:

In analoger Weise wie in Beispiel H-10 beschrieben oder mittels einer entsprechenden anderen der vorstehend angegebenen Verfahrensweisen können auch die in der nachfolgenden Tabelle 12 aufgeführten Verbindungen der allgemeinen Formel Ia.12 hergestellt werden, die z. B. als Ausgangsmaterialen in den Verfahren der Beispiele H-5 dienen.

Tabelle 12:

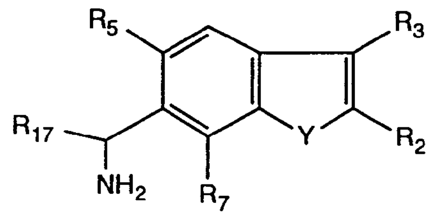

(Ia.12)

| Beispiel | $R_2$ | $R_3$ | $R_5$ | $R_7$ | $R_{17}$ | Y | physikalische Daten |
|---|---|---|---|---|---|---|---|
| H-14.1 | H | H | H | H | Me | S | |
| H-14.2 | H | H | H | H | Et | S | |
| H-14.3 | Me | Me | H | H | Me | O | Oel; $^1$H-NMR |
| H-14.4 | Me | Me | H | H | Et | O | Oel; $^1$H-NMR |
| H-14.5 | H | H | H | Me | Me | O | |
| H-14.6 | H | H | H | Me | Et | O | |
| H-14.7 | H | H | H | Br | Me | O | |
| H-14.8 | H | H | H | Br | Et | O | |
| H-14.9 | H | H | Me | H | Me | O | |
| H-14.10 | H | H | Me | H | Et | O | |
| H-14.11 | Me | Me | H | H | Me | N(H) | Oel; $^1$H-NMR |
| H-14.12 | Me | Me | H | H | Et | N(H) | |
| H-14.13 | Me | Me | H | H | Me | N(Ac) | |
| H-14.14 | Me | Me | H | H | Et | N(Ac) | |
| H-14.15 | Me | Me | H | H | Me | N(Me) | |
| H-14.16 | Me | Me | H | H | Et | N(Me) | |
| H-14.17 | Me | Me | H | H | Me | S | Oel |

Beispiele H-15:

In analoger Weise wie in Beispiel H-10 beschrieben oder mittels einer entsprechenden anderen der vorstehend angegebenen Verfahrensweisen können auch die in der nachfolgenden Tabelle 13 aufgeführten Verbindungen der allgemeinen Formel Ia.13 hergestellt werden, die z. B. als Aüsgangsmaterialien in den Verfahren der Beispiele H-3 dienen.

Tabelle 13:

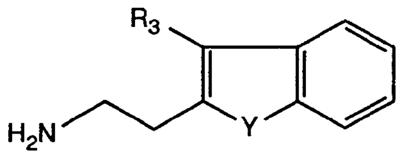

(Ia.13)

| Beispiel | $R_3$ | Y | physikalische Daten |
|---|---|---|---|
| H-15.1 | H | S | |
| H-15.2 | H | O | |
| H-15.3 | H | N(H) | |
| H-15.4 | Me | N(Me) | |

Beispiele H-16:

In analoger Weise wie in Beispiel H-10 beschrieben oder mittels einer entsprechenden anderen der vorstehend angegebenen Verfahrensweisen können auch die in der nachfolgenden Tabelle 14 aufgeführten Verbindungen der allgemeinen Formel Ia.14 hergestellt werden.

Tabelle 14:

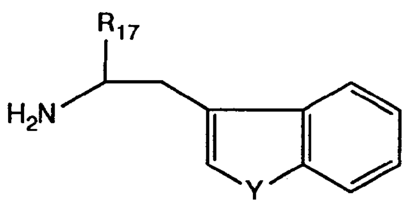

(Ia.14)

| Beispiel | $R_{17}$ | Y | physikalische Daten |
|---|---|---|---|
| H-16.1 | H | S | |
| H-16.2 | H | O | |
| H-16.3 | H | N(Me) | |
| H-16.4 | Me | S | |
| H-16.5 | Me | O | |
| H-16.6 | Me | N(Me) | |
| H-16.7 | Me | NH | |

Beispiele H-17:

In analoger Weise wie in Beispiel H-10 beschrieben oder mittels einer entsprechenden anderen der vorstehend angegebenen Verfahrensweisen können auch die in der nachfolgenden Tabelle 15 aufgeführten Verbindungen der allgemeinen Formel Ia.15 hergestellt werden.

Tabelle 15:

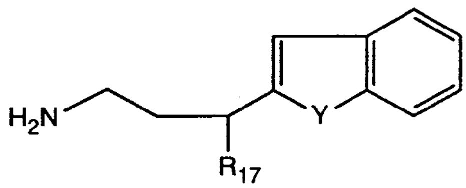

(Ia.15)

| Beispiel | $R_{17}$ | Y | physikalische Daten |
|---|---|---|---|
| H-17.1 | Me | S | |
| H-17.2 | H | S | |
| H-17.3 | Me | O | |
| H-17.4 | H | O | |
| H-17.5 | Me | N(Me) | |
| H-17.6 | H | N(Me) | |

Beispiele H-18:

In analoger Weise wie in Beispiel H-10 beschrieben oder mittels einer entsprechenden anderen der vorstehend angegebenen Verfahrensweisen können auch die in der nachfolgenden Tabelle 16 aufgeführten Verbindungen der allgemeinen Formel Ia. 16 hergestellt werden.

Tabelle 16:

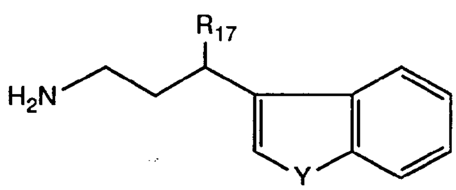

(Ia.16)

| Beispiel | $R_{17}$ | Y | physikalische Daten |
|---|---|---|---|
| H-16.1 | Me | S | |
| H-16.2 | H | S | |
| H-16.3 | Me | O | |
| H-16.4 | H | O | |
| H-16.5 | Me | N(Me) | |
| H-16.6 | H | N(Me) | |

NMR-Daten:

Die nachfolgende Tabelle 17 enthält die $^1$H-NMR-Daten aus vorgenannten Beispielen. Die Aufnahme der $^1$H-NMR-Spektren erfolgte, sofern kein anderes Lösungsmittel angegeben ist, in $CDCl_3$.

Tabelle 17:

| Beispiel | $^1$H-NMR-Daten (ppm/Multiplizität/Anzahl der Protonen) |
|---|---|
| H-2.15 | 8,43/s/1H; 7,77/d/1H; 7,70/d/1H; 7,32/t/1H; 7,29/t/1H; 7,23/s/1H; 5,63/m/1H; 5,58/m/1H; 2,80/q/2H; 2,07/m/2H; 1,27/m/3H; 1,05/t/3H |
| H-2.49 | 8,47/s/1H; 7,67/d/1H; 7,63/d/1H; 7,26/dd/1H; 5,62/q/1H; 5,57/d/1H; 2,78/q/2H; 2,45/s/3H; 2,08/m/1H; 1,95/m/1H; 1,24/t/3H; 1,01/t/3H |
| H-2.62 | 8,40/s/1H; 7,66/d/1H; 7,62/d/1H; 7,25/dd/1H; 5,62/m/1H; 5,57/m/1H; 2,78/q/2H; 2.47/s/3H; 2,08/m/1H; 1.95/m/1H; 1,23/t/3H; 1,01/t/3H |

| | |
|---|---|
| H-5.7 | 8,37/s/1H; 7,35/d/1H; 7,33/d/1H; 7,18/dd/1H; 5,66/d/1H; 5,21/q/1H; 2,77/q/2H; 2,37/s/3H; 2,14/s/3H; 1,97/m/2H; 1,24/t/3H; 0,95/t/3H |
| H-10 | 7,81/d/1H; 7,63/d/1H; 7,36/t/1H; 7,28/t/1H; 4,66/m/1H; 2,37/s/3H; 1,59/s/2H; 1,48/d/3H |
| H-11.1 | 7,80/d/1H; 7,68/d/1H; 7,27/m/3H; 4,43/m/1H; 1,60/s/2H; 1,52/d/3H |
| H-11.2 | 7,80/d/1H; 7,68/d/1H; 7,31/t/1H; 7,27/t/1H; 7,12/s/1H; 4,37/m/1H; 1,82/m/2H; 0,96/t/3H |
| H-11.3 | 7,79/d/1H; 7,64/d/1H; 7,37/t/1H; 7,28/t/1H; 4,40/s/1H; 2,37/s/3H; 1,80/m/2H; 1,58/s/2H; 0,94/t/3H |
| H-11.4 | 7,69/d/1H; 7,59/d/1H; 7,25/dd/1H; 4,66/s/1H; 2,33/s/3H; 1,65/s/2H 1,47/d/3H |
| H-11.5 | 7,70/d/1H; 7,60/s/1H; 7,24/d/1H; 4,33/m/1H; 2,31/s/3H; 1,77/m/2H; 1,60/s/2H; 0,93/t/3H |
| H-11.8 | 7,45/d/1H; 7,33/d/1H; 7,17/dd/1H; 6,42/s/1H; 4,19/m/1H; 1,60/s/2H; 1,45/t/3H |
| H-11.9 | 7,48/d/1H; 7,33/d/1H; 7,17/dd/1H; 6,47/s/1H; 3,96/m/1H; 1,85/m/2H; 1,52/s/2H; 0,96/t/3H |
| H-11.11 | 7,32/d/1H; 6,98/d/1H; 6,83/dd/1H; 3,95/m/1H; 3,84/s/3H; 1,85/m/2H; 1,55/s/2H; 0,97/t/3H |
| H-11.12 | 7,12/m/2H; 6,76/m/1H; 6,49/s/1H; 4,22/s/1H; 4,01/s/3H; 1,62/s/2H; 1,52/d/3H |
| H-11.14 | 7,37/d/1H; 6,99/d/1H; 6,83/dd/1H; 6,41/s/1H; 4,22/s/1H; 3,83/s/3H; 1,82/s/2H; 1,50/d/3H |
| H-11.16 | 6,62/d/1H; 6,48/s/1H; 6,30/d/1H; 4,14/m/1H; 3,88/s/3H; 3,83/s/3H; 1,57/s/2H; 1,48/d/3H |
| H-11.18 | 7,42/m/2H; 7.22/m/2H; 4,30/s/1H; 2,21/s/3H; 1,57/s/2H; 1,49/s(breit)/3H |
| H-11.28 | 7,56/d/1H; 7,28/m/1H; 7,18/t/1H; 7,08/t/1H; 6,40/s/1H; 4,28/q/1H; 3,78/s/3H; 1,55/d/3H; 1,47/s/2H |
| H-11.30 | 7,69/d/1H; 7,60/d/1H; 7,24/dd/1H; 4,67/s/1H; 2,34/s/3H; 1,63/s/2H; 1,47/d/3H |
| H-11.31 | 7,68/d/1H; 7,59/d/1H; 7,23/dd/1H; 4,33/s/1H; 2,32/s/3H; 1,77/m/1H; 1,60/s/2H, 0,92/t/3H |
| H-11.32 | 7,81/d/1H; 7,63/d/1H; 7,36/t/1H; 7,28/t/1H; 4,66/s/1H; 2,37/s/3H; 1,59/s/2H; 1,48/d/3H |
| H-12.21 | 8,00/s/1H; 7,75 bis 7,07/m/5H; 4,50/m/1H; 1,70/s/2H; 1,58/d/3H |
| H-12.26 | 7,68/d/1H; 7,32 bis 7,20/m/2H; 7,11/t/1H; 6,97/s/1H; 4,49/m/1H; 3,75/s/3H; |

| | |
|---|---|
| | 1,56/s/2H; 1,54/d/3H |
| H-14.3 | 7,35/s/1H; 7,33/d/1H; 7,18/dd/1H; 4,21/q/1H; 2,37/s/3H; 2,13/s/3H; 1,53/s/2H; 1,42/d/3H |
| H-14.4 | 7,34/d/1H; 7,32/s/1H; 7,14/dd/1H; 3,88/m/1H; 2,38/s/3H; 2,15/s/3H; 1,72/m/2H; 1,50/s/2H; 0,85/t/3H |
| H-14.11 | 7,72/s/1H; 7,40/d/1H; 7,25/s/1H; 7,05/dd/1H; 4,20/m/1H; 2,38/s/3H; 2,20/s/3H; 1,75 bis 1,31/m/5H |

Beispiele F-1 bis F-6: Formulierung erfindungsgemässer Verbindungen

Beispiele F-1.1 bis F-1.3: Emulsions-Konzentrate

| Bestandteile | F-1.1 | F-1.2 | F-1.3 |
|---|---|---|---|
| Erfindungsgemässer Wirkstoff | 25% | 40% | 50% |
| Calciumdodecylbenzolsulfonat | 5% | 8% | 6% |
| Ricinusölpolyethylenglycolether (36 mol Ethylenoxyeinheiten) | 5% | - | - |
| Tributylphenolpolyethylenglycolether (30 mol Ethylenoxyeinheiten) | - | 12% | 4% |
| Cyclohexanon | - | 15% | 20% |
| Xylolgemisch | 65% | 25% | 20% |

Aus diesen Emulsions-Konzentraten können durch Verdünnung mit Wasser Emulsionen jeder gewünschten Konzentration hergestellt werden.

Beispiel F-2: Emulsions-Konzentrat

| Bestandteile | F-2 |
|---|---|
| Erfindungsgemässer Wirkstoff | 10% |
| Octylphenolpolyethylenglycolether | |

| | |
|---|---|
| (4 bis 5 mol Ethylenoxyeinheiten) | 3% |
| Calciumdodecylbenzolsulfonat | 3% |
| Ricinusölpolyglycolether | |
| (36 mol Ethylenoxyeinheiten) | 4% |
| Cyclohexanon | 30% |
| Xylolgemisch | 50% |

Aus diesem Emulsions-Konzentrat können durch Verdünnung mit Wasser Emulsionen jeder gewünschten Konzentration hergestellt werden.

Beispiele F-3.1 bis F-3.4: Lösungen

| Bestandteile | F-3.1 | F-3.2 | F-3.3 | F-3.4 |
|---|---|---|---|---|
| Erfindungsgemässer Wirkstoff | 80% | 10% | 5% | 95% |
| Propylenglycolmonomethylether | 20% | - | - | - |
| Polyethylenglycol (relative Molekular- | | | | |
| masse: 400 Atommasseneinheiten) | - | 70% | - | - |
| N-Methylpyrrolid-2-on | - | 20% | - | - |
| Epoxidiertes Kokosnussöl | - | - | 1% | 5% |
| Benzin (Siedegrenzen: 160-190°) | - | - | 94% | - |

Die Lösungen sind zur Anwendung in Form kleinster Tropfen geeignet.

Beispiele F-4.1 bis F-4.4: Granulate

| Bestandteile | F-4.1 | F-4.2 | F-4.3 | F-4.4 |
|---|---|---|---|---|
| Erfindungsgemässer Wirkstoff | 5% | 10% | 8% | 21% |
| Kaolin | 94% | - | 79% | 54% |
| Hochdisperse Kieselsäure | 1% | - | 13% | 7% |
| Attapulgit | - | 90% | - | 18% |

Der erfindungsgemässe Wirkstoff wird in Dichlormethan gelöst, die Lösung auf den Träger aufgesprüht und das Lösungsmittel anschliessend im Vakuum abgedampft.

Beispiele F-5.1 und F-5.2: Stäubemittel

| Bestandteile | F-5.1 | F-5.2 |
|---|---|---|
| Erfindungsgemässer Wirkstoff | 2% | 5% |
| Hochdisperse Kieselsäure | 1% | 5% |
| Talkum | 97% | - |
| Kaolin | - | 90% |

Durch inniges Mischen aller Bestandteile erhält man gebrauchsfertige Stäubemittel.

Beispiele F-6.1 bis F-6.3: Spritzpulver

| Bestandteile | F-6.1 | F-6.2 | F-6.3 |
|---|---|---|---|
| Erfindungsgemässer Wirkstoff | 25% | 50% | 75% |
| Natriumligninsulfonat | 5% | 5% | - |
| Natriumlaurylsulfat | 3% | - | 5% |
| Natriumdiisobutylnaphthalinsulfonat | - | 6% | 10% |
| Octylphenolpolyethylenglycolether (7 bis 8 mol Ethylenoxyeinheiten) | - | 2% | - |
| Hochdisperse Kieselsäure | 5% | 10% | 10% |
| Kaolin | 62% | 27% | - |

Alle Bestandteile werden gut gemischt und das Gemisch in einer geeigneten Mühle gut vermahlen. Man erhält Spritzpulver, die sich mit Wasser zu Suspensionen jeder gewünschten Konzentration verdünnen lassen.

Beispiele B-1 bis B-11: Biologische Wirkung erfindungsgemässer Verbindungen

A. Mikrobizide Wirkung

Beispiel B-1.1: Systemische Wirkung gegen Pythium ultimum auf Zuckerrüben

Testmethode: Myzel von Pythium ultimum wird mit Erde gemischt (500 ml Myzelsuspension/10 l Erde) und das Gemisch in 250 ml-Plastikschalen abgefüllt. Nach viertägiger Inkubation bei 10° werden in jede Schale 10 Saatkörner der zu prüfenden Zuckerrübenpflanze gesteckt. Am nächsten Tag werden die Schalen mit je 50 ml einer einen der erfindungsgemässen Wirkstoffe enthaltenden wässrigen Spritzlösung (0,002% Aktivsubstanz) übergossen. Nach einer siebentägigen Inkubationsphase bei 10° und einer anschliessenden viertägigen Inkubationsphase bei 22° wird die Wirkung der Aktivsubstanz nach Zahl und Aussehen der aufgelaufenen Pflanzen bewertet.
Testresultat: Erfindungsgemässe Wirkstoffe zeigen gegen pythium ultimum auf Zuckerrüben gute systemische Wirkung.

Beispiel B-1.2:Systemische Wirkung gegen Pythium ultimum auf Mais

Testmethode: Der Test wird in analoger Weise wie in Beispiel B-1.1 beschrieben durchgeführt.
Testresultat: Erfindungsgemässe Wirkstoffe zeigen gegen Pythium ultimum auf Mais gute systemische Wirkung.

Beispiel B-2: Wirkung gegen Puccinia graminis auf Weizen

a) Residual-protektive Wirkung

Testmethode: Weizenpflanzen werden 6 Tage nach Aussaat mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. Die Beurteilung der Rostpustelnentwicklung 12 Tage nach Infektion führt zur Bewertung der Wirkung der Aktivsubstanz.
Testresultat: Erfindungsgemässe Wirkstoffe zeigen gegen Puccinia graminis auf Weizen gute residual-protektive Wirkung, z.B. reduzieren die Wirkstoffe gemäss Beispielen H-1, H-2.13, H-2.15 und H-2.18 den Pilzbefall auf 20 bis 5%. Nicht mit der Aktivsubstanz behandelte infizierte Kontrollpflanzen weisen dagegen einen Pilzbefall von 100% auf.

b) Systemische Wirkung

Testmethode: Zu Weizenpflanzen wird 5 Tage nach Aussaat eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. Die Beurteilung der Rostpustelnentwicklung 12 Tage nach Infektion führt zur Bewertung der Wirkung der Aktivsubstanz.
Testresultat: Erfindungsgemässe Wirkstoffe gemäss den Beispielen H-1, H-2.13, H-2.15 und H-2.18 zeigen gegen Puccinia graminis auf Weizen gute systemische Wirkung.

Beispiel B-3: Wirkung gegen Phytophthora infestans auf Tomaten

a) Residual-protektive Wirkung

Testmethode: Tomatenpflanzen werden nach dreiwöchiger Anzucht mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Sporangiensuspension des Pilzes infiziert. Die Beurteilung des Pilzbefalls 5 Tage nach Infektion, während denen 90 bis 100 Prozent relative Luftfeuchtigkeit und eine Temperatur von 20° aufrechterhalten werden, führt zur Bewertung der Wirkung der Aktivsubstanz.
Testresultat: Erfindungsgemässe Wirkstoffe zeigen gegen Phytophthora infestans auf Tomaten gute residual-protektive Wirkung, z. B. reduzieren die Wirkstoffe gemäss den Beispielen H-2.15, H-2.18 und H-2.29 den Pilzbefall auf 5 bis 0%. Nicht mit der Aktivsubstanz behandelte infizierte Kontrollpflanzen weisen dagegen einen Pilzbefall von 100% auf.

b) Systemische Wirkung

Testmethode: Zu Tomatenpflanzen wird nach dreiwöchiger Anzucht eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. Nach 48 Stunden werden die Pflanzen mit einer Sporangiensuspension des Pilzes infiziert. Die Beurteilung des Pilzbefalls 5 Tage nach Infektion, während denen 90 bis 100 Prozent relative Luftfeuchtigkeit und eine Temperatur von 20° aufrechterhalten werden, führt zur Bewertung der Wirkung der Aktivsubstanz.
Testresultat: Erfindungsgemässe Wirkstoffe zeigen gegen Phytophthora infestans auf Tomaten gute systemische Wirkung.

Beispiel B-4: Residual-protektive Wirkung gegen Cercospora arachidicola auf Erdnüssen

Testmethode: 10 bis 15 cm hohe Erdnusspflanzen werden mit einer aus einem einen der erfindungsgemässen Wirkstoffe enthaltenden Spritzpulver hergestellten wässrigen Spritzbrühe (0,02% Aktivsubstanz) tropfnass besprüht und 48 Stunden später mit einer Konidiensuspension des Pilzes inkubiert. Die Pflanzen werden 72 Stunder bei 21° und hoher Luftfeuchtigkeit inkubiert und anschliessend bis zum Auftreten der typischen Blattflecken in einem Gewächshaus aufgestellt. Die Bewertung der Wirkung der Aktivsubstanz erfolgt 12 Tage nach Infektion aufgrund von Anzahl und Grösse der Blattflecken.
Testresultat: Erfindungsgemässe Wirkstoffe zeigen gegen Cercospora arachidicola auf Erdnüssen gute residual-protektive Wirkung.

Beispiel B-5: Wirkung gegen Plasmopara viticola auf Reben

a) Residual-protektive Wirkung

Testmethode: Rebensämlinge im 4 bis 5 Blatt-Stadium werden mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Sporangiensuspension des Pilzes infiziert. Die Beurteilung des Pilzbefalls 6 Tage nach Infektion, während denen 95 bis 100 Prozent relative Luftfeuchtigkeit und eine Temperatur von 20° aufrechterhalten werden, führt zur Bewertung der Wirkung der Aktivsubstanz.
Testresultat: Erfindungsgemässe Wirkstoffe zeigen gegen Plasmopara viticola auf Reben gute präventive residual-protektive Wirkung, z. B. reduziert der Wirkstoff gemäss Beispiel H-2.29 den Pilzbefall auf 20 bis 0%. Nicht mit der Aktivsubstanz behandelte infizierte Kontrollpflanzen weisen dagegen einen Pilzbefall von 100% auf.

b) Residual-protektive Wirkung

Testmethode: Rebensämlinge im 4 bis 5 Blatt-Stadium werden mit einer Sporangiensuspension des Pilzes infiziert, 24 Stunden in einer Feuchtkammer (Bewdingungen: 95 bis 100 Prozent relative Luftfeuchtigkeit bei 20°) inkubiert und dann mit einer aus einem einen der erfindungsgemässen Wirkstoffe enthaltenden Spritzpulver hergestellten wässrigen Spritzbrühe (0,02% Aktivsubstanz) tropfnass besprüht. Nach Antrocknen des Spritzbelags werden die Pflanzen wieder in die Feuchtkammer gebracht. Die Beurteilung des Pilzbefalls 6 Tage nach Infektion führt zur Bewertung der Wirkung der Aktivsubstanz.
Testresultat: Erfindungsgemässe Wirkstoffe zeigen gegen Plasmopara viticola auf Reben gute kurative residual-protektive Wirkung.

Beispiel B-6: Wirkung gegen Pyricularia oryzae auf Reis

a) Residual-protektive Wirkung

Testmethode: Reispflanzen werden nach zweiwöchiger Anzucht mit einer aus einem einen der erfindungsgemässen Wirkstoffe enthaltenden Spritzpulver hergestellten wässrigen Spritzbrühe (0,02% Aktivsubstanz) tropfnass besprüht und 48 Stunden später mit einer Konidiensuspension des Pilzes infiziert. Die Beurteilung des Pilzbefalls 5 Tage nach Infektion, während denen 95 bis 100 Prozent relative Luftfeuchtigkeit und eine Temperatur von 22° aufrechterhalten werden, führt zur Bewertung der Wirkung der Aktivsubstanz.
Testresultat: Erfindungsgemässe Wirkstoffe zeigen gegen Pyricularia oryzae auf Reis gute residual-protektive Wirkung.

b) Systemische Wirkung

Testmethode: Zu 2 Wochen alten Reispflanzen wird eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet dass die Spritzbrühe nicht mit oberirdischen Pflanzenteilen in Berührung kommt. Dann werden die Töpfe so weit mit Wasser gefüllt, dass die untersten Stengelteile der Reispflanzen im Wasser stehen. Nach 96 Stunden werden die Pflanzen mit einer Konidiensuspension des Pilzes infiziert. Die Beurteilung des Pilzbefalls 5 Tage nach Infektion, während denen 95 bis 100 Prozent relative Luftfeuchtigkeit und eine Temperatur von 24° aufrechterhalten werden, führt zur Bewertung der Wirkung der Aktivsubstanz.

Testresultat: Erfindungsgemässe Wirkstoffe zeigen gegen Pyricularia oryzae auf Reis gute systemische Wirkung.

Beispiel B-7: Residual-protektive Wirkung gegen Venturia inaequalis auf Aepfeln

Testmethode: Apfelstecklinge mit 10 bis 20 cm langen Frischtrieben werden mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Konidiensuspension des Pilzes infiziert. Die Pflanzen werden 5 Tage bei 90 bis 100 Prozent relativer Luftfeuchtigkeit inkubiert und weitere 10 Tage in einem Gewächshaus bei 20 bis 24° aufgestellt. Die Beurteilung des Schorfbefalls 15 Tage nach Infektion führt zur Bewertung der Wirkung der Aktivsubstanz.
Testresultat: Erfindungsgemässe Wirkstoffe gemäss den Beispielen H-1 und den Tabellen 1, 2 und 4, insbesondere H-2.13, H-2.15, H-2.18, H-2.19, H-2.26, H-2.27, H-2.39, H-2.61, H-2.65, H-2.68, H-3.1, H-3.13, H-5.6 und H-5.7 zeigen gegen Venturia inaequalis auf Aepfeln gute residual-protektive Wirkung.

Beispiel B-8: Wirkung gegen Erysiphe graminis auf Gerste

a) Residual-protektive Wirkung

Testmethode: Ungefähr 8 cm hohe Gerstenpflanzen werden mit einer aus einem einen der erfindungsgemässen Wirkstoffe enthaltenden Spritzpulver hergestellten wässrigen Spritzbrühe (0,02% Aktivsubstanz) tropfnass besprüht und 3 bis 4 Stunden später mit Konidien des Pilzes bestäubt. Die infizierten Pflanzen werden in einem Gewächshaus bei 22° aufgestellt. Die Beurteilung des Pilzbefalls 10 Tage nach Infektion führt zur Bewertung der Wirkung der Aktivsubstanz.
Testresultat: Erfindungsgemässe Wirkstoffe zeigen gegen Erysiphe graminis auf Gerste gute residual-protektive Wirkung, z. B. reduzieren die Wirkstoffe gemäss den Beispielen H-2.18 und H-2.29 den Pilzbefall auf 5 bis 0%. Nicht mit der Aktivsubstanz behandelte infizierte Kontrollpflanzen weisen dagegen einen Pilzbefall von 100% auf.

b) Systemische Wirkung

Testmethode: Zu ungefähr 8 cm hohen Gerstenpflanzen wird eine aus einem einen der erfindungsgemässen Wirkstoffe enthaltenden Spritzpulver hergestellte wässrige Spritzbrühe (0,002% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit Konidien des Pilzes bestäubt. Die infizierten Pflanzen werden in einem Gewächshaus bei 22° aufgestellt. Die Beurteilung des Pilzbefalls 10 Tage nach Infektion führt zur Bewertung der Wirkung der Aktivsubstanz.
Testresultat: Erfindungsgemässe Wirkstoffe zeigen gegen Erysiphe graminis auf Gerste gute systemische Wirkung.

Beispiel B-9: Wirkung gegen Podosphaera leucotricha auf Apfeltrieben Residual-protektive Wirkung

Apfelstecklinge mit ca. 15 cm langen Frischtrieben werden mit einer Spritzbrühe (0,06 % Aktivsubstanz) besprüht. Nach 24 Stunden werden die behandelten Pflanzen mit einer Konidiensuspension des Pilzes infiziert und in einer Klimakammer bei 70 % relativer Luftfeuchtigkeit und 20°C aufgestellt. Die Beurteilung des Pilzbefalls erfolgt 12 Tage nach der Infektion.
Mit Wirkstoffen der Formel I aus den Tabellen 1, 2 und 4 wird der Krankheitsbefall auf unter 20 % verhütet. Kontrollpflanzen sind zu 100 % befallen.

B. Akarizide Wirkung

Beispiel B-10: Wirkung gegen Tetranychus urticae auf Bohnen

Testmethode: Junge Bohnenpflanzen werden mit einer Mischpopulation von Tetranychus urticae besiedelt, einen Tag später mit einer aus einem einen der erfindungsgemässen Wirkstoffe enthaltenden Spritzpulver hergestellten wässrigen Emulsions-Spritzbrühe (0,04% Aktivsubstanz) tropfnass besprüht und anschliessend 6 Tage bei 25° inkubiert. Die Wirkung der Aktivsubstanz wird sodann auf der Basis einer Auszählung der Schädlinge bewertet. Dabei werden jeweils die toten Eier, toten Larven und toten Adulte an den behandelten Pflanzen

gezählt und in analoger Weise die entsprechenden Zahlenwerte an nicht mit der Aktivsubstanz behandelten Kontrollpflanzen ermittelt Aus den jeweiligen Wertepaaren für die behandelten und die unbehandelten Pflanzen wird der Prozentsatz berechnet, um welchen die Schädlingspopulation an den behandelten Pflanzen reduziert worden ist (Prozent Wirkung der Aktivsubstanz).
Testresultat: Erfindungsgemässe Wirkstoffe zeigen gegen Tetranychus urticae auf Bohnen gute Wirkung.

Beispiel B-11: Wirkung gegen Tetranychus cinnabarinus auf Buschbohnen

Testmethode (Verdünnungsreihe): Buschbohnen im 2 Blatt-Stadium werden mit einer Mischpopulation (Eier, Larve/Nymphen und Adulte) eines OP-toleranten Stammes von Tetranychus cinnabarinus besiedelt. Der erfindungsgemässe Wirkstoff wird 24 Stunden später in Konzentrationen von 200, 100 und 50 mg/l in der automatischen Spritzkabine auf die Pflanzen appliziert (der Wirkstoff ist formuliert und wird jeweils mit Wasser bis zu der entsprechenden Konzentration verdünnt). Die Wirkung der Aktivsubstanz wird 2 und 7 Tage nach der Applikation auf der Basis einer Auszählung der Schädlinge bewertet. Dabei werden jeweils die toten Eier, toten Larven/Nymphen und toten Adulte an den (mit Wirkstoff in der jeweiligen Konzentration) behandelten Pflanzen gezählt und in analoger Weise die entsprechenden Zahlenwerte an nicht mit der Aktivsubstanz behandelten Kontrollpflanzen ermittelt. Aus den jeweiligen Wertepaaren für die (mit Wirkstoff in der jeweiligen Konzentration) behandelten und die unbehandelten Pflanzen wird der Prozentsatz berechnet, um welchen die Schädlingspopulation an den behandelten Pflanzen bei der jeweiligen Wirkstoffkonzentration reduziert worden ist (Prozent Wirkung der Aktivsubstanz).
Testresultat: Erfindungsgemässe Wirkstoffe zeigen gegen Tetranychus cinnabarinus auf Buschbohnen gute Wirkung.

## Patentansprüche

1. Eine Verbindung der Formel

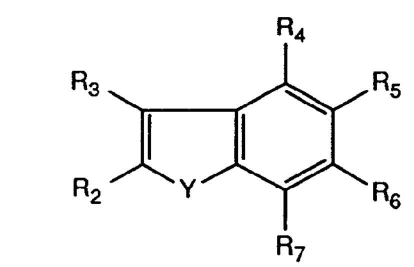

(I),

worin bedeuten:
einer der Reste $R_2$, $R_3$, $R_4$, $R_5$, $R_6$ und $R_7$ eine Gruppe der Formel

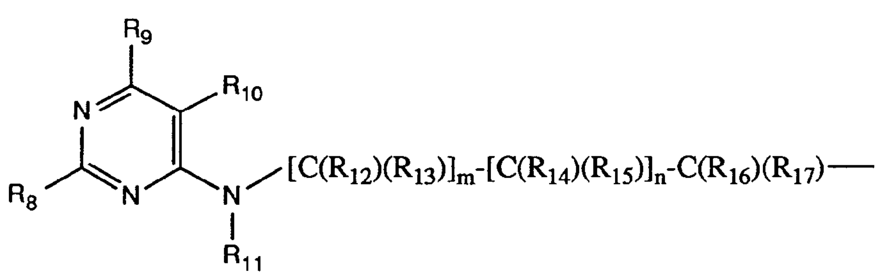

(Ia);

die anderen fünf der Reste $R_2$, $R_3$, $R_4$, $R_5$, $R_5$ und $R_7$, unabhängig voneinander, Wasserstoff, Halogen, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, Halogen-$C_1$-$C_8$-alkoxy, $C_1$-$C_8$-Alkylthio Halogen-$C_1$-$C_8$-alkylthio, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_4$-Alkyl und $C_1$-$C_4$-Alkoxy, substituiert sind, mit der Massgabe, dass von den fünf Resten aus der Gruppe der Reste $R_2$, $R_3$, $R_4$, $R_5$, $R_5$ und $R_7$, die von der Gruppe Ia verschieden sind, mindestens zwei Reste für Wasserstoff stehen und mindestens vier Reste

von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind;
Y ein Sauerstoffatom, ein Schwefelatom oder eine Gruppe der Formel $NR_1$ (Ib);
$R_1$ Wasserstoff, $C_1$-$C_8$-Alkyl, $C_1$-$C_8$-Alkanoyl, $C_1$-$C_8$-Alkansulfonyl, Halogen-$C_1$-$C_8$-alkansulfonyl, Cyano-$C_1$-$C_8$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_4$-Alkyl, Nitro und Cyano, substituiert ist;
$R_8$ Wasserstoff, $C_1$-$C_8$-Alkyl, $C_1$-$C_8$-Alkoxy oder $C_1$-$C_8$-Alkylthio;
$R_9$ Wasserstoff, $C_1$-$C_8$-Alkyl, Halogen-$C_1$-$C_8$-alkyl, $C_1$-$C_8$-Alkoxy-$C_1$-$C_4$-alkyl, $C_1$-$C_4$-Alkylthio-$C_1$-$C_4$-alkyl, $C_1$-$C_4$-Alkansulfinyl-$C_1$-$C_4$-alkyl, $C_1$-$C_4$-Alkansulfonyl-$C_1$-$C_4$-alkyl, $C_2$-$C_8$-Alkenyl, Halogen-$C_2$-$C_8$-alkenyl, $C_2$-$C_8$-Alkinyl, $C_3$-$C_8$-Cycloalkyl oder Halogen;
$R_{10}$ Wasserstoff, Hydroxy, $C_1$-$C_8$-Alkyl, Halogen-$C_1$-$C_8$-alkyl, $C_1$-$C_4$-Alkoxy-$C_1$ -$C_4$-alkyl, $C_1$-$C_8$-Alkoxy, $C_1$-$C_8$-Alkylthio, $C_1$-$C_8$-Alkansulfinyl, $C_1$-$C_8$-Alkansulfonyl, Halogen, Nitro, Cyano, Amino, eine Gruppe der Formel $N(H)R_{18}$ (Ic), eine Gruppe der Formel $N(R_{18})R_{19}$ (Id) oder eine Gruppe der Formel $N{=}C(R_{19})R_{20}$ (Ie);
$R_{11}$ Wasserstoff, $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_4$-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind;
$R_{12}$, $R_{13}$, $R_{14}$, $R_{15}$, $R_{16}$ und $R_{17}$, unabhängig voneinander, Wasserstoff, $C_1$-$C_8$-Alkyl, Halogen-$C_1$-$C_8$-alkyl, $C_1$-$C_4$-Alkoxy-$C_1$-$C_4$-alkyl oder $C_3$-$C_8$-Cycloalkyl;
$R_{18}$ $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_4$-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind;
$R_{19}$ $C_1$-$C_8$-Alkyl;
$R_{20}$ Wasserstoff oder $C_1$-$C_8$-Alkyl;
m 0 oder 1; und
n 0 oder 1;
oder gegebenenfalls ein Tautomeres davon, jeweils in freier Form oder in Salzform.

2. Eine Verbindung gemäss Anspruch 1 der Formel I, worin bedeuten:
einer der Reste $R_2$, $R_3$, $R_4$, $R_5$, $R_6$ und $R_7$ eine Gruppe Ia;
die anderen fünf der Reste $R_2$, $R_3$, $R_4$, $R_5$, $R_6$ und $R_7$, unabhängig voneinander, Wasserstoff, Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_4$-Alkoxy, Halogen-$C_1$-$C_4$-alkoxy mit 1, 2 oder 3 Halogenatomen, $C_1$-$C_4$-Alkylthio, Halogen-$C_1$-$C_4$-alkylthio mit 1, 2 oder 3 Halogenatomen, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_2$-Alkyl und $C_1$-$C_2$-Alkoxy, substituiert sind, mit der Massgabe, dass von den fünf Resten aus der Gruppe der Reste $R_2$, $R_3$, $R_4$, $R_5$, $R_6$ und $R_7$, die von der Gruppe Ia verschieden sind, mindestens zwei Reste für Wasserstoff stehen und mindestens vier Reste von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind;
Y ein Sauerstoffatom, ein Schwefelatom oder eine Gruppe Ib;
$R_1$ Wasserstoff, $C_1$-$C_4$-Alkyl, $C_1$-$C_4$-Alkanoyl, $C_1$-$C_4$-Alkansulfonyl, Halogen-$C_1$-$C_4$-alkansulfonyl mit 1, 2 oder 3 Halogenatomen, Cyano-$C_1$-$C_4$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;
$R_8$ Wasserstoff, $C_1$-$C_4$-Alkyl, $C_1$-$C_4$-Alkoxy oder $C_1$-$C_4$-Alkylthio;
$R_9$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$ -$C_4$-alkyl, $C_1$-$C_2$-Alkansulfonyl-$C_1$-$C_4$-alkyl, $C_2$-$C_4$-Alkenyl, Halogen-$C_2$-$C_4$-alkenyl mit 1, 2 oder 3 Halogenatomen, $C_2$-$C_4$- Alkinyl, $C_3$-$C_7$-Cycloalkyl oder

Halogen;
$R_{10}$ Wasserstoff, Hydroxy, $C_1$-$C_4$-Alkyl, Halogen-$C_1$-$C_4$-alkyl mit 1, 2 oder 3 Halogenatomen, $C_1$-$C_4$-Alkoxy-$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro, Cyano, Amino, eine Gruppe Ic, eine Gruppe Id oder eine Gruppe Ie;
$R_{11}$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_{12}$, $R_{13}$, $R_{14}$, $R_{15}$, $R_{16}$ und $R_{17}$, unabhängig voneinander, Wasserstoff, $C_1$-$C_4$-Alkyl ,Halogen-$C_1$-$C_4$-alkyl mit 1, 2 oder 3 Halogenatomen, $C_1$-$C_4$-Alkoxy-$C_1$-$C_4$-alkyl oder $C_3$-$C_7$-Cycloalkyl;
$R_{18}$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-Alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_{19}$ $C_1$-$C_4$-Alkyl;
$R_{20}$ Wasserstoff oder $C_1$-$C_4$-Alkyl;
m 0 oder 1; und
n 0 oder 1;
oder gegebenenfalls ein Tautomeres davon, jeweils in freier Form oder in Salzform.

3. Eine Verbindung gemäss Anspruch 1 der Formel I, worin bedeuten:
einer der Reste $R_2$, $R_3$, $R_5$ und $R_5$ eine Gruppe Ia;
die anderen drei der Reste $R_2$, $R_3$, $R_5$ und $R_5$ und die Reste $R_4$ und $R_7$, unabhängig voneinander, Wässerstoff, Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_4$-Alkoxy, Halogen-$C_1$-$C_4$-alkoxy mit 1, 2 oder 3 Halogenatomen, $C_1$-$C_4$-Alkylthio, Halogen-$C_1$-$C_4$-alkylthio mit 1, 2 oder 3 Halogenatomen, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_2$-Alkyl und $C_1$-$C_2$-Alkoxy, substituiert sind, mit der Massgabe, dass von den fünf Resten aus der Gruppe der Reste $R_2$, $R_3$, $R_4$, $R_5$, $R_6$ und $R_7$, die von der Gruppe Ia verschieden sind, mindestens zwei Reste für Wasserstoff stehen und mindestens vier Reste von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind;
Y ein Sauerstoffatom, ein Schwefelatom oder eine Gruppe Ib;
$R_1$ Wasserstoff, $C_1$-$C_4$-Alkyl, $C_1$-$C_4$-Alkanoyl, $C_1$-$C_4$-Alkansulfonyl, Halogen-$C_1$-$C_4$-alkansulfonyl mit 1, 2 oder 3 Halogenatomen, Cyano-$C_1$-$C_4$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;
$R_8$ Wasserstoff, $C_1$-$C_4$-Alkyl, $C_1$-$C_4$-Alkoxy oder $C_1$-$C_4$-Alkylthio;
$R_9$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$-$C_4$-alkyl, $C_1$-$C_2$-Alkansulfonyl-$C_1$-$C_4$-alkyl, $C_2$-$C_4$-Alkenyl, Halogen-$C_2$-$C_4$-alkenyl mit 1, 2 oder 3 Halogenatomen, $C_2$-$C_4$-Alkinyl, $C_3$-$C_7$-Cycloalkyl oder Halogen;
$R_{10}$ Wasserstoff, Hydroxy, $C_1$-$C_4$-Alkyl, Halogen-$C_1$-$C_4$-alkyl mit 1, 2 oder 3 Halogenatomen, $C_1$-$C_4$-Alkoxy-$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro, Cyano, Amino, eine Gruppe Ic, eine Gruppe Id oder eine Gruppe Ie;
$R_{11}$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_{12}$, $R_{13}$, $R_{14}$, $R_{15}$, $R_{16}$ und $R_{17}$, unabhängig voneinander, Wasserstoff, $C_1$-$C_4$-Alkyl, Halogen-$C_1$-$C_4$-alkyl mit 1, 2 oder 3 Halogenatomen, $C_1$-$C_4$-Alkoxy-$C_1$-$C_4$-alkyl oder $C_1$-$C_4$-Cycloalkyl;

$R_{18}$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_{19}$ $C_1$-$C_4$-Alkyl;
$R_{20}$ Wasserstoff oder $C_1$-$C_4$-Alkyl;
m 0 oder 1; und
n 0 oder 1;
oder gegebenenfalls ein Tautomeres davon, jeweils in freier Form oder in Salzform.

**4.** Eine Verbindung gemäss Anspruch 1 der Formel I, worin bedeuten:
einer der Reste $R_2$, $R_3$, $R_5$ und $R_5$ eine Gruppe Ia;
die anderen drei der Reste $R_2$, $R_3$, $R_5$ und $R_6$ und die Reste $R_4$ und $R_7$, unabhängig voneinander, Wasserstoff, Halogen, $C_1$-$C_4$-Alkyl, $C_1$-$C_4$-Alkoxy oder unsubstituiertes Phenyl, mit der Massgabe, dass von den fünf Resten aus der Gruppe der Reste $R_2$, $R_3$, $R_4$, $R_5$, $R_6$ und $R_7$, die von der Gruppe Ia verschieden sind, mindestens zwei Reste für Wasserstoff stehen und mindestens vier Reste von unsubstituiertem Phenyl verschieden sind;
Y ein Sauerstoffatom, ein Schwefelatom oder eine Gruppe Ib;
$R_1$ Wasserstoff, $C_1$-$C_4$-Alkyl, $C_1$-$C_4$-Alkanoyl oder unsubstituiertes Phenylsulfonyl;
$R_8$ Wasserstoff oder $C_1$-$C_4$-Alkyl;
$R_9$ $C_1$-$C_4$-Alkyl, $C_3$-$C_4$-Cycloalkyl oder Halogen;
$R_{10}$ $C_1$-$C_4$-Alkyl, Halogen oder Nitro;
$R_{11}$, $R_{12}$, $R_{13}$, $R_{14}$, $R_{15}$ und $R_{16}$ jeweils Wasserstoff;
$R_{17}$ Wasserstoff oder $C_1$-$C_4$-Alkyl;
m 0 oder 1; und
n 0 oder 1;
in freier Form oder in Salzform.

**5.** Eine Verbindung gemäss Anspruch 1 der Formel I, worin bedeuten:
einer der Reste $R_2$, $R_3$ und $R_6$ eine Gruppe Ia;
die beiden anderen der Reste $R_2$, $R_3$ und $R_6$ und die Reste $R_4$, $R_5$ und $R_7$, unabhängig voneinander, Wasserstoff, Halogen, $C_1$-$C_4$-Alkyl oder $C_1$-$C_4$-Alkoxy, mit der Massgabe, dass von den fünf Resten aus der Gruppe der Reste $R_2$, $R_3$, $R_4$, $R_5$, $R_6$ und $R_7$, die von der Gruppe Ia verschieden sind, mindestens drei Reste für Wasserstoff stehen; Y ein Sauerstoffatom, ein Schwefelatom oder eine Gruppe Ib; $R_1$ Wasserstoff oder $C_1$-$C_4$-Alkyl;
$R_8$ Wasserstoff;
$R_9$ $C_1$-$C_4$-Alkyl;
$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

**6.** Eine Verbindung gemäss Anspruch 1 der Formel I, worin bedeuten:
(a) $R_2$ eine Gruppe Ia, $R_3$ Wasserstoff oder $C_1$-$C_4$-Alkyl, $R_4$ Wasserstoff, $R_5$ Wasserstoff, Halogen oder $C_1$-$C_4$-Alkoxy, $R_5$ und $R_7$ jeweils Wasserstoff, mit der Massgabe, dass von den fünf Resten $R_3$, $R_4$, $R_5$, $R_6$ und $R_7$ mindestens vier Reste für Wasserstoff stehen, Y ein Sauerstoffatom, ein Schwefelatom oder eine Gruppe Ib und $R_1$ Wasserstoff oder $C_1$-$C_4$-Alkyl; oder
(b) $R_2$ Wasserstoff, $R_3$ eine Gruppe Ia, $R_4$, $R_5$, $R_6$ und $R_7$ jeweils Wasserstoff, Y eine Gruppe Ib und $R_1$ Wasserstoff oder $C_1$-$C_4$-Alkyl; oder
(c) $R_2$ und $R_3$, unabhängig voneinander, $C_1$-$C_4$-Alkyl, $R_4$ und $R_5$ jeweils Wasserstoff, $R_6$ eine Gruppe Ia, $R_7$ Wasserstoff, Y ein Sauerstoffatom oder eine Gruppe Ib und $R_1$ Wasserstoff; sowie jeweils
$R_8$ Wasserstoff;
$R_9$ $C_1$-$C_4$-Alkyl;
$R_{10}$ Halogen;
$R_{11}$ und $R_{16}$ jeweils Wasserstoff;

$R_{17}$ $C_1$-$C_4$-Alkyl; und
m und n jeweils 0;
in freier Form oder in Salzform.

7. Eine Verbindung gemäss Anspruch 1 der Formel I, worin bedeuten:
(d) $R_2$ eine Gruppe Ia, $R_6$ Wasserstoff und entweder $R_3$ Wasserstoff oder $C_1$-$C_4$-Alkyl, $R_5$ Wasserstoff und Y ein Schwefelatom oder $R_3$ Wasserstoff, $R_5$ Halogen oder $C_1$-$C_4$-Alkoxy und Y ein Sauerstoffatom; oder
(e) $R_2$ und $R_3$, unabhängig voneinander, $C_1$-$C_4$-Alkyl, $R_5$ Wasserstoff, $R_6$ eine Gruppe Ia und Y ein Sauerstoffatom; sowie jeweils
$R_4$, $R_7$ und $R_6$ jeweils Wasserstoff;
$R_9$ $C_1$-$C_4$-Alkyl;
$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

8. Eine Verbindung gemäss Anspruch 1 der Formel I, worin bedeuten:
entweder $R_3$ $C_1$-$C_4$-Alkyl, $R_6$ Wasserstoff und Y ein Schwefelatom oder $R_3$ Wasserstoff, $R_5$ $C_1$-$C_4$-Alkoxy und Y ein Sauerstoffatom; sowie jeweils
$R_2$ eine Gruppe Ia;
$R_4$, $R_6$, $R_7$ und $R_6$ jeweils Wasserstoff;
$R_9$ $C_1$-$C_4$-Alkyl
$R_{10}$ Halogen;
$R_{11}$ und $R_{16}$ jeweils Wasserstoff;
$R_{17}$ $C_1$-$C_4$-Alkyl; und
m und n jeweils 0;
in freier Form oder in Salzform.

9. Eine Verbindung gemäss Anspruch 1 der Formel I, worin bedeuten:
Y ein Schwefelatom;
$R_2$ eine Gruppe Ia;
$R_3$ $C_1$-$C_4$-Alkyl;
$R_4$, $R_6$, $R_6$, $R_7$ und $R_8$ jeweils Wasserstoff;
$R_9$ $C_1$-$C_4$-Alkyl;
$R_{10}$ Halogen;
$R_{11}$ und $R_{16}$ jeweils Wässerstoff;
$R_{17}$ $C_1$-$C_4$-Alkyl; und
m und n jeweils 0;
in freier Form oder in Salzform.

10. Eine Verbindung aus der Gruppe
(d,l)-2-[1-(5-Chlor-6-ethyl-pyrimidin-4-ylamino)ethyl]-3-methyl-benzo[b]thiophen, (Bsp. H-2.18)
(d,l)-2-[1-(5-Chlor-6-ethyl-pyrimidin-4-ylamino)ethyl]-5-methoxy-benzo[b]furan, (Bsp. H-2.29)
jeweils in freier Form oder in Salzform.

11. Eine Verbindung aus der Gruppe
(d,l)-2-[1-(5-Chlor-6-ethyl-pyrimidin-4-ylamino)ethyl]-benzo[b]thiophen,(Bsp.H-2.13)
(d,l)-2-[1-(5-Chlor-6-ethyl-pyrimidin-4-ylamino)propyl]-benzo[b]thiophen,(Bsp.H-2.15)
(-)-2-[1-(5-Chlor-6-ethyl-pyrimidin-4-ylamino)propyl]-benzo[b]thiophen,
jeweils in freier Form oder in Salzform.

12. Verfahren zur Herstellung einer Verbindung der Formel I oder gegebenenfalls eines Tautomeren davon, jeweils in freier Form oder in Salzform, dadurch gekennzeichnet, dass man
a) zur Herstellung einer Verbindung der Formel I, worin $R_{11}$ Wasserstoff, $C_1$-$C_8$-Alkyl oder Benzyl ist, oder gegebenenfalls eines Tautomeren davon, jeweils in freier Form oder in Salzform, eine Verbindung der Formel

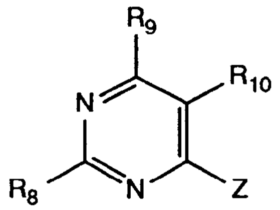

(II),

worin $R_8$, $R_9$ und $R_{10}$ die für die Formel I angegebenen Bedeutungen haben und Z ein leicht abspaltbarer nucleofuger Rest ist, oder gegebenenfalls ein Tautomeres davon mit einer Verbindung der Formel

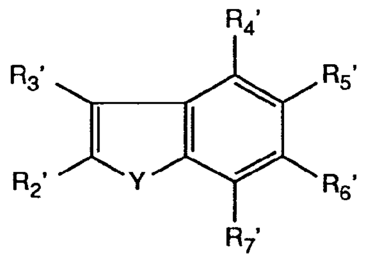

(III),

worin Y die Formel die Formel I angegebene Bedeutung hat und mit einer einzigen Ausnahme $R_2'$ für $R_2$, $R_3'$ für $R_3$, $R_4'$ für $R_4$, $R_5'$ für $R_5$, $R_5'$ für $R_5$ und $R_7'$ für $R_7$, wobei $R_2$, $R_3$, $R_4$, $R_5$, $R_6$ und $R_7$ die für die Formel I angegebenen Bedeutungen haben, steht, wobei die erwähnte Ausnahme darin besteht, dass anstelle der in den Definitionen der Variablen $R_2$, $R_3$, $R_4$, $R_5$, $R_6$ und $R_7$ der Formel I erwähnten Gruppe Ia die Gruppe der Formel $H(R_{11}')N-[C(R_{12}(R_{13})]_m-[C(R_{14})R_{15}]_n-C(R_{16})(R_{17})-$ (IIIa) steht, worin $R_{11}'$ Wasserstoff, $C_1$-$C_8$-Alkyl oder Benzyl ist und $R_{12}$, $R_{13}$, $R_{14}$, $R_{15}$, $R_{16}$, $R_{17}$, m und n die für die Formel I angegebenen Bedeutungen haben, oder einem Salz davon, vorzugsweise in Gegenwart einer Base, umsetzt oder

b) zur Herstellung einer Verbindung der Formel I, worin $R_{11}$ die für die Formel I angegebene Bedeutung hat, jedoch von Wasserstoff, $C_1$-$C_8$-Alkyl und Benzyl verschieden ist, oder gegebenenfalls eines Tautomeren davon, jeweils in freier Form oder in Salzform, in eine Verbindung der Formel I, worin $R_{11}$ Wasserstoff ist, oder gegebenenfalls ein Tautomeres davon, jeweils in freier Form oder in Salzform, den gewünschten, von Wasserstoff, $C_1$-$C_8$-Alkyl und Benzyl verschiedenen, Substituenten $R_{11}$ durch N-Alkanoylierung, N-Benzoylierung, N-Alkylthiolierung, N-Phenylthiolierung oder N-Benzylthiolierung einführt

und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

**13.** Mittel zum Schutz von Pflanzen gegen den Befall durch Schädlinge, dadurch gekennzeichnet, dass es als Wirkstoff mindestens eine Verbindung gemäss Anspruch 1 oder gegebenenfalls ein Tautomeres davon, jeweils in freier Form oder in agrochemisch verwendbarer Salzform, enthält, gegebenenfalls neben mindestens einem Hilfsstoff.

**14.** Mittel gemäss Anspruch 13 zum Schutz von Pflanzen gegen den Befall durch phytopathogene Mikroorganismen und/oder Schädlinge der Ordnung Akarina.

**15.** Verfahren zur Herstellung eines Mittels gemäss Anspruch 13, dadurch gekennzeichnet, dass man den Wirkstoff mit dem Trägermaterial innig vermischt.

**16.** Verwendung einer Verbindung gemäss Anspruch 1 oder gegebenenfalls eines Tautomeren davon, jeweils in freier Form oder in agrochemisch verwendbarer Salzform, oder eines Mittels gemäss Anspruch 13 zum Schutz von Pflanzen gegen den Befall durch Schädlinge.

**17.** Verwendung gemäss Anspruch 16 zum Schutz von Pflanzen gegen den Befall durch phytopathogene Mikroorganismen und/oder Schädlinge der Ordnung Akarina.

**18.** Verfahren zum Schutz von Pflanzen gegen den Befall durch Schädlinge, dadurch gekennzeichnet, dass man als Wirkstoff eine Verbindung gemäss Anspruch 1 oder gegebenenfalls ein Tautomeres davon, jeweils in freier Form oder in agrochemisch verwendbarer Salzform, auf die Pflanzen, deren Standort oder auf Pflanzenteile appliziert.

**19.** Verfahren gemäss Anspruch 18 zum Schutz von Pflanzen gegen den Befall durch phytopathogene Mikroorganismen und/oder Schädlinge der Ordnung Akarina.

**20.** Eine Verbindung der Formel IV

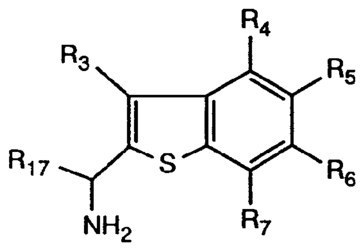

IV

worin bedeuten
$R_3$, $R_4$, $R_5$, $R_6$ und $R_7$, unabhängig voneinander, Wasserstoff, Halogen, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, Halogen-$C_1$-$C_8$-alkoxy, $C_1$-$C_8$-Alkylthio, Halogen-$C_1$-$C_8$-Alkylthio, Cyano, Nitro, Phenyl, Phenoxy oder Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_4$-Alkyl und $C_1$-$C_4$-Alkoxy, substituiert sind, mit der Massgabe, dass von den fünf Resten $R_3$, $R_4$, $R_5$, $R_6$ und $R_7$, mindestens zwei Reste für Wasserstoff stehen und mindestens vier Reste von gegebenenfalls wie vorstehend erwähnt substituiertem Phenyl, von gegebenenfalls wie vorstehend erwähnt substituiertem Phenoxy und von gegebenenfalls wie vorstehend erwähnt substituiertem Phenylthio verschieden sind;
$R_{17}$ Ethyl;
in freier Form oder in Salzform.

**21.** Eine Verbindung gemäss Anspruch 20, worin bedeuten:
$R_3$, $R_4$, $R_5$, $R_6$ und $R_7$ unabhängig voneinander Wasserstoff, Methyl, Ethyl, Methoxy oder Chlor;
$R_{17}$ Ethyl;
in freier Form oder in Salzform.

**22.** Eine Verbindung gemäss Anspruch 21 aus der Gruppe
(d,l)-2-(1-Aminopropyl)-benzo[b]thiophen;
(-)-2-(1-Aminopropyl)-benzo[b]thiophen;
jeweils in freier Form oder in Salzform.

Europäisches Patentamt

# EUROPÄISCHER RECHERCHENBERICHT

Nummer der Anmeldung

EP 92 81 0641

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | WO-A-9 208 704 (E. I. DU PONT DE NEMOURS) 29. Mai 1992 * Seite 120 - Seite 121; Ansprüche * | 1,10-19 | C07D403/12 C07D405/12 C07D409/12 C07D333/58 C07D333/62 A01N43/54 |
| A | EP-A-0 130 735 (AMERICAN HOME PRODUCTS) * Seite 18; Ansprüche; Beispiel 2 * | 1 | |
| A | FR-A-2 100 663 (ASPRO-NICHOLAS) * Seite 1 - Seite 13 * | 20-22 | |
| | | | RECHERCHIERTE SACHGEBIETE (Int. Cl.5) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 07 DEZEMBER 1992 | FRANCOIS J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)